# EUROPEAN PATENT SPECIFICATION

(11) **EP 3 570 897 B1**
(45) Date of publication and mention of the grant of the patent: **04.10.2023**
(21) Application number: 18700616.8
(22) Date of filing: 23.01.2018
(51) Int. Cl.: A61K 51/10, A61P 43/00

(54) **RADIOIMMUNOCONJUGATE FOR USE IN TREATING BONE MARROW ASSOCIATED DISEASES**
RADIOIMMUNKONJUGATE ZUR VERWENDUNG BEI DER BEHANDLUNG VON KRANKHEITEN IM ZUSAMMENHANG MIT KNOCHENMARK
RADIOIMMUNOCONJUGUÉ DESTINÉ À ÊTRE UTILISÉ DANS LE TRAITEMENT DE MALADIES ASSOCIÉES À LA MOELLE OSSEUSE

(30) Priority: 23.01.2017 EP 17152694
(43) Date of publication of application: 27.11.2019
(73) Proprietor: TheraPharm GmbH, 6340 Baar (CH)
(72) Inventor: ORCHARD, Kim, Old Bursledon Hampshire SO31 8DH (GB); BOSSLET, Klaus, 82377 Penzberg (DE)
(74) Representative: Weickmann & Weickmann PartmbB
(86) International application number: PCT/EP2018/051533
(87) International publication number: WO 2018/134430

(56) References cited:
- WO-A1-2011/144744
- US-A1- 2012 070 440
- PRICE ERIC W ET AL: "A comparative evaluation of the chelators H4octapa and CHX-A''-DTPA with the therapeutic radiometal90Y", NUCLEAR MEDICINE AND BIOLOGY, ELSEVIER, NY, US, vol. 43, no. 9, 28 June 2016 (2016-06-28), pages 566-576, XP029688784, ISSN: 0969-8051, DOI: 10.1016/J.NUCMEDBIO.2016.06.004
- CAMERA L ET AL: "EVALUATION OF THE SERUM STABILITY AND IN VIVO BIODISTRIBUTION OF CHX-DTPA AND OTHER LIGANDS FOR YTTRIUM LABELING OF MONOCLONAL ANTIBODIES", THE JOURNAL OF NUCLEAR MEDICINE, SOCIETY OF NUCLEAR MEDICINE, US, vol. 35, no. 5, 1 May 1994 (1994-05-01), pages 882-889, XP008065955, ISSN: 0161-5505
- SAROSIEK SHAYNA ET AL: "Comparing measures of hematologic response after high-dose melphalan and stem cell transplantation in AL amyloidosis", BLOOD CANCER JOURNAL, vol. 10, no. 8, 1 January 2020 (2020-01-01), page 88, XP93033678, DOI: 10.1038/s41408-020-00354-7 Retrieved from the Internet: URL:https://www.nature.com/articles/s41408 -020-00354-7.pdf>

## Description

The present invention relates to the use of radioimmunoconjugates for the treatment of bone marrow associated diseases, particularly AL-amyloidosis.

### Background of the invention

The bone marrow is the flexible tissue in the interior of bones. It is responsible for the production of red blood cells, white blood cells, myelocytes, plasma cells and platelets. The bone marrow contains stem cells that are primitive cells capable of turning into any desired cell in the body. As needed, the stem cells differentiate to become a particular kind of cell. From the bone marrow, only the mature cells are released into the blood stream.

Bone marrow associated diseases are usually linked to an abnormality in the production of any of the mature blood cells or the precursor or predecessor immature cells.

In one embodiment of the invention, bone marrow associated diseases are those that can be treated by stem cell transplantation, e.g. autologous stem cell transplantation or allogeneic stem cell transplantation. In an aspect of the invention bone marrow associated diseases are those that can be treated by hematopoietic stem cell transplantation. Stem cell transplantation, in particular hematopoietic stem cell transplantation, is used in certain diseases to substitute for a defect, e.g. at the metabolism level, immunity level, gene level or malignant transformation of the bone marrow.

In AL-amyloidosis, abnormal neoplastic clones of plasma cells in the bone marrow overproduce monoclonal immunoglobulin light chains which self-assemble and deposit in organs, causing organ dysfunction. The deposits have a characteristic β-pleated secondary structure seen by electron microscopy as 8-10 nm linear non-branching fibrils. Virtually any organ or combination of organs other than the brain may be affected.

AL-amyloidosis is diagnosed by congo red staining of a tissue biopsy specimen that shows pathognomonic apple green dichroism under polarized light. Recently, radiolabeled serum amyloid P scintigraphy (SAP) was developed at the UK National Amyloidosis Center as a tool for non-invasive imaging and monitoring of amyloid deposits and has unequivocally demonstrated that amyloid deposits of all types exist in a state of dynamic turnover and may regress when the abundance of the respective fibril precursor protein is sufficiently reduced.

AL-amyloidosis has a lifetime incidence and is the cause of death of between 0.5-1 per a thousand individuals in the UK. It occurs equally in men and women and the median age at diagnosis is 65 years. Without therapy, it is inexorably progressive and until recently, the median survival was only 6-15 months.

After the immunoglobulin nature of AL-amyloidosis deposits was established, case reports claiming beneficial responses to cytotoxic therapy appeared in the literature. Efficacy of oral melphalan and prednisolone (MP) was systematically compared to colchicine, effectively a placebo, in a prospective randomized trial of 219 patients by the Mayo Clinic group. Median survival was 17 months and 8.5 months in the MP and colchicine groups, respectively, demonstrating robustly for the first time the benefit of cytotoxic therapy in AL-amyloidosis (Kyle et al., The New England journal of medicine, 1997; 336(17): 1202-7). In an attempt to identify which patients with AL-amyloidosis were likely to derive the greatest benefit from therapy with MP, the Mayo Clinic investigators reported long-term follow-up of 153 patients who received this therapy for a planned 24-36 months (Gertz et al., Journal of clinical oncology, 1999; 17(1): 262-7). Using stringent response criteria, which included complete disappearance of any pre-treatment serum or urine monoclonal protein in conjunction with improvement in organ dysfunction, 18% of the patients responded. The median survival among responders was 89.4 months and the median time to achieve a response was 11.7 months. However, one quarter of the responders died of myelodysplasia or acute leukemia.

The relatively poor and delayed responses to MP in AL-amyloidosis prompted interest in the use of high-dose melphalan therapy and stem cell transplantation (ASCT). This was first reported in 1996 and a series of 25 patients was reported shortly afterwards by Comenzo and colleagues (Comenzo et al., Blood, 1998; 91(10): 3662-70). Several centers subsequently reported clinical benefit in about 60% of patients who survived the procedure. However, treatment-related mortality was substantially and consistently higher among patients with amyloid than those with myeloma. The 100-day mortality in two experienced single-center US studies was around 14% and in two multi-center European studies -40%, reflecting compromised function of multiple organ systems by amyloid. These findings highlighted the need for refinement in patient selection for ASCT and improvement in peri-transplant clinical management whilst also prompting interest in alternative, less toxic, therapeutic regimens.

In the US, patient selection was refined (Comenzo et al., Blood, 2002; 99(12): 4276-82) and ASCT remained the therapeutic *"gold-standard"* for treating AL-amyloidosis. Results of long-term follow-up of 701 consecutive patients with systemic AL-amyloidosis were recently reported by the Boston Group; 394 (56%) were considered eligible for ASCT, 82 of whom did not proceed because of patient choice or disease progression (Skinner et al., Ann Intern Med, 2004; 140(2): 85-93). Treatment-Related Mortality (TRM) among 312 patients who initiated therapy was 13% and median survival for the whole cohort was 4.6 years. Median survival among ineligible patients was only 4 months. Interestingly, Dispenzieri et al. examined data from patients with AL-amyloidosis treated at the Mayo clinic from 1983 to 1997 and identified 229 patients who would now have been eligible for ASCT. Their median survival was 42 months and 5- and 10-year survival rates were 36% and 15%, respectively, suggesting that the survival benefit with ASCT is, in part, due to patient selection rather than the specific therapeutic regimen. A French randomized trial cast further doubts on the role of ASCT in AL-amyloidosis due to high treatment-related mortality of ~22% and no improvement in overall survival in the ASCT arm over oral melphalan dexamethasone chemotherapy in a landmark analysis (Jaccard et al., The New England journal of medicine, 2007; 357(11): 1083-93). However, the high TRM and lower than expected responses lead to much criticism of this study. The role of ASCT therefore remains unclear and practice in the UK National Amyloidosis Centre has recently been to rarely recommend ASCT as first line therapy, but to consider its role in patients who have not responded adequately to first line therapy with intermediate dose chemotherapy regimens such as vincristine, adriamycin and dexamethasone (VAD). The outcome among all 92 patients with AL-amyloidosis who were seen at the UK National Amyloidosis Centre and underwent ASCT between 1994 and 2004 was recently determined, the median overall survival for the whole cohort was 63 months and although TRM was 23%, retrospective analysis identified a subset of patients with no TRM (Goodman et al., HaematologicalThe Hematology Journal, 2005; 90(s1): 201). Factors identified from the NAC study lead to a refinement of patient selection criteria in the UK and data from 2003-2011 from 83 patients undergoing ASCT revealed a TRM of 6%.

Even in a highly selected patient group, TRM and morbidity of the treatment persists. The majority of the treatment morbidity is due to the toxicity of high-dose melphalan leading to mucositis, end organ damage due to the drug or complication of cytopenias.

High-dose melphalan treatment has to be applied before patients receive their own hematopoietic stem cells (autologous transplantation; ASCT) or the hematopoietic stem cells of genetically related human donors (allogeneic transplantation). Such conditioning is the key step to eliminating both the hematopoietic system of the patient as well as the malignant stem cells to prepare the patient for a successful engraftment and to generate optimal therapeutic effects.

Due to the toxicity of the high-dose melphalan conditioning step, ASCT can only be applied to eligible patients who are in relatively good physical state, mainly younger patients at the age of less than 65 years and/or those with good organ function.

Despite the careful selection of so-called eligible patients, there still is a single digit percentage of treatment-related mortality (TRM) mainly caused by the various toxicities of high-dose melphalan treatment and the direct or indirect consequences. Clearly, there is an unabated need for selective methods of treatment that make it possible to reduce the treatment-related mortality mainly caused by the high-dose melphalan treatment.

A number of radioimmunoconjugates (RIC) using monoclonal antibodies selective for certain antigens, such as CD45, CD33, CD20, CD19 and CD66, have been the subject of investigation for bone marrow conditioning before transplantation in hematological malignancies, such as acute lymphoblastic leukemia (ALL), acute myeloid leukemia (AML) and transformed myelodysplasia (MDS) (Matthews D. et al., Blood, 1999, 94: 1237-1247; Jurcic JG, Cancer Biother Radiopharm., 2000, 15: 319-326; Bunjes D. et al., Blood, 2001, 98: 565-572). Radioimmunoconjugates were applied in addition to standard conditioning regimens. However, most of these radioimmunoconjugates show a non-selective uptake in the organs. The cause of this non-target uptake of a radioimmunoconjugate is multifactorial and includes specific and non-specific uptake and instability of the immunoconjugate *in vivo.* The known RICs show severe dose-limiting toxicity in liver, lung and kidney, due to selectivity of the antibodies used and/or the stability of the attached radiolabel (e.g. as observed using the ¹⁸⁸Re labelled molecule of anti-CD66 mAb studied by Bunjes et al., Blood, 2001, 98: 565-572). Unfortunately, only a marginal part of the injected dose reaches the desired target (Karger, Contrib. Oncol. Basel, 1992, 43, 100-145). This limitation is the major reason for many unsuccessful clinical investigations in the targeted antibody field, wherein dose-limiting toxicity did not allow generating the desired therapeutic effects.

WO 2007/062855 describes the treatment of multiple myeloma using a CD66 monoclonal antibody radiolabeled with ⁹⁰Y. The described radioimmunoconjugate does not accumulate in liver or kidney and selectively targets marrow and spleen. In a first step, dosimetry studies with ¹¹¹In-labelled anti-CD66 radioimmunoconjugates were preformed to determine the respective patient suitability for therapy. Those patients suffering from multiple myeloma who showed a favorable dosimetry (more than 90%) were conditioned with ⁹⁰Y-labelled anti-CD66 (stepwise from 5 MBq/kg lbw to 45 MBq/kg lbw). After this first conditioning step, the patients received the standard high-dose melphalan treatment followed by autologous hematopoietic stem cell transplantation.

Results from a randomized clinical phase II study show that anti-CD66 antibody targeted radiotherapy (ATRT) followed by high-dose melphalan and ASCT (arm A) in comparison to high-dose melphalan and ASCT (arm B) show a complete regression rate from 50%, whereas arm B had a complete regression rate of only 25%. Side-effect profiles of both treatment arms were comparable.

WO 2011/144744 suggests targeted radioimmunoconjugates (RIC) in combination with cytotoxic agents, such as melphalan, following stem cell transplantation for treating inflammatory and/or autoimmune disorders.

US 2012/070440 A1 refers to a conjugate compound comprising a monoclonal antibody or antigen binding fragment that binds to a human CS1 epitope and inhibits immunoglobulin secretion, linked to an effector moiety, e.g. selected from a cytotoxic agent, a detection moiety, an activatable moiety, a chemotherapeutic agent, a lipase, an antibiotic, a chemoattracting moiety, an immune modulator, or a radioisotope, or to a detectable label, e.g. selected from a radioactive compound, a fluorescent compound, an enzyme, a substrate, an epitope tag or a toxin.

The inventors have unexpectedly found that targeted radioimmunotherapy in bone marrow conditioning using a RIC comprising a CD66-binding component and a radionuclide is suitable for use in treating patients, particularly human patients, suffering from bone marrow associated diseases such as AL-amyloidosis without the occurrence of detectable side-effects.

Thus, one aspect of the present invention is a radioimmunoconjugate (RIC), which comprises a CD66-binding component and a therapeutically effective radionuclide for use in treating bone marrow associated diseases, namely AL-amyloidosis, wherein the CD66-binding component is an antibody, and wherein the radionuclide is linked to the CD66-binding component via a chelating agent, which is covalently linked to the CD66-binding component.

That is, according to the present invention, a radioimmunoconjugate (RIC) that comprises a CD66-binding component and a radionuclide can be used as a medicament for administration in a therapy for bone marrow associated diseases, namely AL-amyloidosis.

In another aspect, radioimmunoconjugates comprising a CD66-binding component and a therapeutically effective radionuclide are for use as a medicament in bone marrow conditioning, particularly preceding stem cell transplantation, more particularly hematopoietic stem cell transplantation, wherein the CD66-binding component is an antibody, wherein the radionuclide is linked to the CD66-binding component via a chelating agent, which is covalently linked to the CD66-binding component and wherein the therapeutic RIC is for administration to a patient who is ineligible to treatment with high dose-melphalan preceding HSCT and/or to a patient who was treated with induction therapy but had disease progression. Bone marrow conditioning can comprise immunoablation and myeloablation to prevent graft rejection and/or reducing the number of defective cells in the bone marrow.

Bone marrow conditioning can e.g. be measured by determining the depletion of the bone marrow using histopathological staining procedures known in the art or by evaluating the degrees of peripheral blood cell counts. Alternatively, the acceptance of the transplanted cells may be evaluated as a measure of successful conditioning.

In a preferred embodiment, the subsequent stem cell transplantation may be autologous or allogeneic. It was shown that the bone marrow conditioning according to the invention does not cause any side-effects, such as nausea and vomiting, fever, mucositis, diarrhea and sepsis, which usually occur in prior art bone marrow conditioning. The use of RIC according to the invention in bone marrow conditioning thus reduces or even avoids in-patient treatment as compared to bone marrow conditioning treatment according to prior art regimens, such as high-dose chemotherapies, e.g. high-dose melphalan treatments. In a preferred embodiment RICs of the invention are preferably for use as a medicament in bone marrow conditioning without additional cytotoxic or cytostatic agents, such as melphalan.

In another embodiment, RICs of the invention are for use as a medicament in bone marrow conditioning together with a reduced dose of cytotoxic or cytostatic agents as compared to standard prior art doses.

The treatment regimen comprises administration of radionuclides suitable for therapeutic irradiation and optionally imaging of bone marrow. The RIC administration preferably is a conditioning regimen in combination with further therapeutic measures, particularly stem cell transplantation. The radionuclide of the RIC is a therapeutically effective radionuclide. For example, the therapeutically effective radionuclide may be a β- or β/γ-emitting radionuclide, such as yttrium-90 (⁹⁰Y), iodine-131 (¹³¹I), samarium-153 (¹⁵³Sm), holmium-166 (¹⁶⁶Ho), rhenium-186 (¹⁸⁶Re), rhenium-188 (¹⁸⁸Re) or lutetium-177 (¹⁷⁷Lu) or may be an α-emitter such as astatine-211 (²¹¹At), bismuth-212 (²¹²Bi), bismuth-213 (²¹³Bi) or actinium-225 (²²⁵Ac). In a preferred embodiment, the therapeutically effective radionuclide is yttrium-90 (⁹⁰Y).

An imaging radionuclide is a radionuclide which is suitable for monitoring and/or determining pharmacokinetics of the RIC. For example, the imaging radionuclide may be indium-111 (¹¹¹In), iodine-131 (¹³¹I) or technetium-99m (^{99m}Tc). In a preferred embodiment, the imaging radionuclide is indium-111 (¹¹¹In).

In a particularly preferred embodiment, the RIC of the invention is for use as a medicament encompassing determining the therapeutically effective dose of the therapeutic RIC prior to administration. This determination may be carried out individually for a subject to be treated or for a group of subjects, e.g. based on the severity of progression of the disease. For example, the RIC of the invention may be for use as a medicament comprising the administration of a RIC comprising an imaging radionuclide and a subsequent administration of the RIC comprising a therapeutically effective radionuclide. By means of first administering an imaging RIC, the effective dose of the subsequently administered therapeutic RIC may be individually determined and/or adjusted for the respective subject, e.g. a human patient. In this embodiment, the CD66-binding component of the imaging RIC and the therapeutic RIC is preferably identical, at least with respect to the CD66 binding specificity and/or affinity.

In a preferred embodiment, the imaging RIC is administered to a patient 2-10 days, preferably 2-8 days, prior to administering a therapeutic RIC.

It should be noted, however, that administration of an imaging RIC prior to administration of a therapeutic RIC might not be necessary if sufficient patient data has been collected, e.g. in a database, to determine a therapeutically active amount of the RIC. Thus, a further preferred embodiment of the invention comprises an RIC for use as a medicament comprising determining a therapeutically effective dose of the RIC by evaluating pre-existing data, e.g. from a database, particularly combined with immunohistochemical analysis of the patient's bone marrow cellularity.

The CD66-binding component is an antibody, particularly a monoclonal antibody, a chimeric antibody, a humanized antibody or a recombinant antibody, such as a single-chain antibody or fragment thereof, e.g. proteolytic antibody fragments such as Fab-, Fab'- or F(ab)₂-fragments or recombinant antibody fragments such as single-chain Fv-fragments.

The CD66-binding component may also be a fusion polypeptide comprising at least one antibody-binding domain, namely an antibody, and a further domain, e.g. an effector domain, such as an enzyme or cytokine.

In a preferred embodiment, the CD66-binding component selectively binds to the human CD66 antigen or an epitope thereof, e.g. CD66a, b, c or e. In a particularly preferred embodiment, the CD66-binding component is the BW250/183 antibody. Murine, humanized and recombinant forms of this antibody are described in EP-A-0 388 914, EP-A-0 585 570 and EP-A-0 972 528.

The radionuclide is linked to the CD66-binding component via a chelating agent, with the linkage being a covalent linkage. Preferably, the radionuclide is linked to the CD66-binding component via a structure of the formula

[(chelating agent)-(R¹)ₚ-(R²-R³)ₙ]ₘ-(CD66-binding component)

wherein n is 0 or 1,
m is 1 to 15,
p is 0 or 1,
R¹ and R³ are independently selected from the group consisting of -NH-CS-NH-, -NHCONH-, -NHCOCH₂S-, -S-S-, -NH-NH-, -NH-, -S-, -CONHNH-, -SCH₂CH₂COONH-, -SCH₂CH₂SO₂-, -SCH₂CH₂SO₂NH-, -CONH-, -O-CH₂CH₂O-, -CO-, -COO-, -NH-O-, -CONHO-, -S-(CH₂)₃C(NH)NH-, -NH-COO-, -O- and 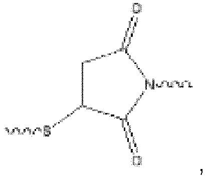
preferably -NHCSNH-, and
R² is selected from the group consisting of C1-C18 alkylen, branched C1-C18, -CH₂-C₆H₁₀-, p-alkylphenylene, p-phenylene, m-phenylene, p-alkyloxyphenylene, naphthylene, -[CH₂CH₂O]ₓ-, -[CH₂CH₂SOCH₂CH₂]ₓ-, -[CH₂CH₂SO₂CH₂CH₂]ₓ-, or -[NHCHR₄CO]_{y}-, wherein x is 1 to 200, y is 1 to 20, and wherein R₄ is selected from the group consisting of H-, Me-, HSCH₂-, isopropyl, but-2-yl, CH₃SCH₂CH₂-, benzyl, 1*H*-indol-3-yl-methyl, HOCH₂-, HOOCCH₂-, CH₃CH(OH)-, HOOCCH₂CH₂-, 4-hydroxybenzyl, H₂NCOCH₂-, H₂NCOCH₂CH₂-, 4-aminobut-1-yl, 2-guanidinoethyl, 1*H*-imidazol-5-yl-methyl and 2-methylprop-1-yl.

For example, the chelating agent may be selected from the group consisting of diethylenetriaminepentaacetic acid (DTPA), 1,4,7,10-tetraazacyclododecane-N,N',N",N‴-tetraacetic acid (DOTA), 1,4,8,11-tetraazacyclotetradecane-N,N',N",N‴-tetraacetic acid (TETA), 1,4,7-triazonane-N,N',N"-triacetic acid (NOTA), 2,2'-(2-(((1S,2S)-2-(bis(carboxymethyl)amino)cyclohexyl)-(carboxymethyl)amino)ethylazanediyl)diacetic acid (cyclohexano-DTPA), 2,2'-(2-(((1R,2R)-2-(bis(carboxymethyl)amino)cyclohexyl)-(carboxymethyl)amino)ethylazanediyl)diacetic acid, 2,2'-(2-(((1S,2R)-2-(bis(carboxymethyl)amino)cyclohexyl)-(carboxymethyl)amino)ethylazanediyl)diacetic acid, 2,2'-(2-(((1R,2S)-2-(bis(carboxymethyl)amino)cyclohexyl)-(carboxymethyl)amino)ethylazanediyl)diacetic acid, 2,2',2",2‴-(2,2'-(1S,2S)-cyclohexane-1,2-diylbis((carboxymethyl)azanediyl)bis(ethane-2,1-diyl))bis(azanetriyl)tetraacetic acid, 2,2',2",2‴-(2,2'-(1S,2R)-cyclohexane-1,2-diylbis((carboxymethyl)azanediyl)bis(ethane-2,1-diyl))bis(azanetriyl)tetraacetic acid, (1*R*)-1-benzyl-diethylenetriaminepentaacetic acid, (1*S*)-1-benzyl-diethylenetriaminepentaacetic acid, (2*R*)-2-benzyl-diethylenetriaminepentaacetic acid, (2*S*)-2-benzyl-diethylenetriaminepentaacetic acid, (2*R*)-2-benzyl-(3R)-3-methyl-DTPA, (2R)-2-benzyl-(3*S*)-3-methyl-DTPA, (2*S*)-2-benzyl-(3*S*)-3-methyl-DTPA, (2*S*)-2-benzyl-(3*R*)-3-methyl-DTPA, (2*R*)-2-benzyl-(4*R*)-4-methyl-DTPA, (2*R*)-2-benzyl-(4*S*)-4-methyl-DTPA, (2*S*)-2-benzyl-(4*S*)-4-methyl-DTPA, (2*S*)-2-benzyl-(4*R*)-4-methyl-DTPA, (1*R*)-1-benzyl-(3*R*)-3-methyl-DTPA, (1*R*)-1-benzyl-(3*S*)-3-methyl-DTPA, (1*S*)-1-benzyl-(3*S*)-3-methyl-DTPA, (1*S*)-1-benzyl-(3*R*)-3-methyl-DTPA, (1*R*)-1-benzyl-(4*R*)-4-methyl-DTPA, (1*R*)-1-benzyl-(4*S*)-4-methyl-DTPA, (1*S*)-1-benzyl-(4*S*)-4-methyl-DTPA, (1*S*)-1-benzyl-(4*R*)-4-methyl-DTPA, 2,2'-((1R,2R)-2-(((R)-2-(bis(carboxymethyl)amino)-3-phenylpropyl)(carboxymethyl)amino)cyclohexylazanediyl)diacetic acid, 2,2'-((1S,2S)-2-(((S)-2-(bis(carboxymethyl)amino)-3-phenylpropyl)(carboxymethyl)amino)cyclohexylazanediyl)diacetic acid, 2,2'-((1R,2R)-2-(((S)-2-(bis(carboxymethyl)amino)-3-phenylpropyl)(carboxymethyl)amino)cyclohexylazanediyl)diacetic acid, 2,2'-((1S,2S)-2-(((R)-2-(bis(carboxymethyl)amino)-3-phenylpropyl)(carboxymethyl)amino)cyclohexylazanediyl)diacetic acid, 2,2'-((1R,2S)-2-(((R)-2-(bis(carboxymethyl)amino)-3-phenylpropyl)(carboxymethyl)amino)cyclohexylazanediyl)diacetic acid, 2,2'-((1S,2R)-2-(((S)-2-(bis(carboxymethyl)amino)-3-phenylpropyl)(carboxymethyl)amino)cyclohexylazanediyl)diacetic acid, 2,2'-((1S,2R)-2-(((R)-2-(bis(carboxymethyl)amino)-3-phenylpropyl)(carboxymethyl)amino)cyclohexylazanediyl)diacetic acid, 2,2'-((1R,2S)-2-(((S)-2-(bis(carboxymethyl)amino)-3-phenylpropyl)(carboxymethyl)amino)cyclohexylazanediyl)diacetic acid, (2S)-2-benzyl-1,4,7,10-tetraazacyclododecane-N,N',N",N‴-tetraacetic acid, (2R)-2-benzyl-1 ,4,7,1 0-tetraazacyclododecane-N, N',N",N‴-tetraacetic acid, 6-benzyl-1,4,8,11-tetraazacyclotetradecane-N,N',N",N‴-tetraacetic acid, 2-benzyl-1,4,7-triazonane-N,N',N"-triacetic acid, benzyl-3-methyl-diethylenetriaminepentaacetic acid (2B3M-DTPA), (R)-2-amino-3-(phenyl)propyl)trans-(S,S)-cyclohexane-1,2-diamine-pentaacetic acid) (Bn-CHX-A"-DTPA, also designated as CHX-A"-DTPA) and salts and derivatives thereof,
particularly (R)-2-amino-3-(phenyl)propyl)trans-(S,S)-cyclohexane-1,2-diamine-pentaacetic acid) (Bn-CHX-A"-DTPA, also designated as CHX-A"-DTPA) and salts thereof.

A preferred radioimmunoconjugate for use according to the invention has the structure CD66-binding component-NH-CS-NH-Bn-CHX-A"-DTPA, also designated as CD66-binding component-NH-CS-NH-CHX-A"-DTPA, and salts (e.g. chloride salt) thereof according to formula I

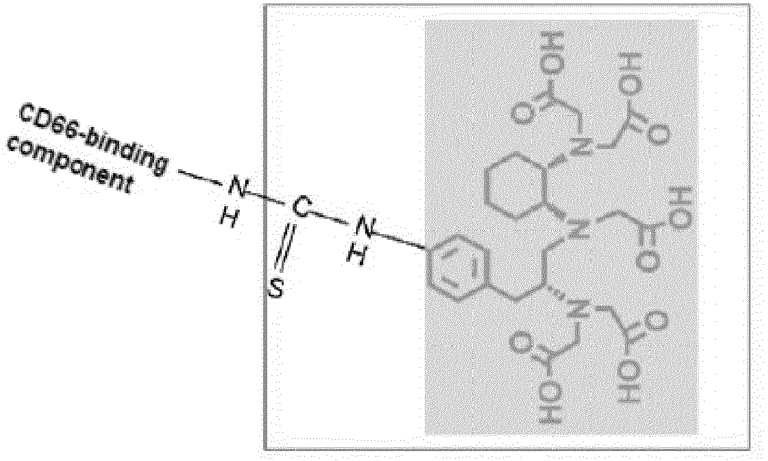

. More preferably, the radioimmunoconjugate is a BW250/183-NH-CS-NH-Bn-CHX-A"-DTPA (also designated as BW250/183-NH-CS-NH-CHX-A"-DTPA) or a salt thereof.

The administration of the therapeutic RIC for the treatment of human patients is preferably in a dose of ≥ about 10 MBq/kg lean body weight (lbw), preferably ≥ about 15 MBq/kg lbw, more preferably ≥ about 20 MBq/kg lbw, still more preferably ≥ about 25 MBq/kg bw, still more preferably ≥ about 30 MBq/kg lbw and still more preferably ≥ about 35 MBq/kg lbw. Preferably, the dose of the therapeutic radioimmunoconjugate ranges up to 60 MBq/kg lbw, preferably up to 50 MBq/kg lbw, more preferably up to 45 MBq/kg lbw.

The RIC may be administered according to known methods, e.g. by infusion, optionally together with pharmaceutically acceptable carriers.

The RIC for use according to the invention is preferably administered as conditioning regimen in a therapy that comprises additional measures, such as stem cell transplantation, particularly autologous or allogeneic stem cell transplantation, more particularly autologous stem cell transplantation. The therapeutic radioimmunoconjugate is preferably administered to the patient 6-16 days, preferably 6-12 days, before stem cell transplantation.

In a further preferred embodiment, the treatment of bone marrow associated diseases, such as AL-amyloidosis, according to the present invention does not involve administration of an additional anti-tumor agent, such as melphalan, or an immunosuppressive agent at least four weeks before and/or after stem cell transplantation.

In a further preferred embodiment, the therapeutic RIC for use in the treatment of bone marrow associated diseases, such as AL-amyloidosis, according to the present invention is for administration to a patient who was treated with high-dose melphalan followed by stem cell transplantation but had disease progression.

In a further preferred embodiment, the therapeutic RIC for use in the treatment of bone marrow associated diseases, such as AL-amyloidosis, according to the present invention is for administration to a patient, who was treated with induction therapy e.g. velcade, dexamethasone, thalidomide; or velcade, dexamethasone, cyclophosphamide; or velcade, dexamethasone; or melphalan, prednisone; or pomalidomide, dexamethasone) but had disease progression.

In a further preferred embodiment, the therapeutic RIC for use in the treatment of bone marrow associated diseases, such as AL-amyloidosis, according to the present invention is for administration to a patient, who is ineligible for HD-melphalan preceding HSCT.

Therapeutic protocols, particularly for the therapy of bone marrow associated diseases such as AL amyloidosis, may comprise the steps:
(a) optionally administering an imaging RIC to the patient,
(b) administering a therapeutic RIC to the patient and
(c) transplanting autologous or allogeneic stem cells.

Therapeutic protocols, particularly for the therapy of bone marrow associated diseases such as AL amyloidosis, may consist of the steps:
(a) optionally administering an imaging RIC to the patient,
(b) administering a therapeutic RIC to the patient,
(c) optionally administering antibiotics, and
(d) transplanting autologous or allogeneic stem cells.

Therapeutic protocols, particularly for the therapy of bone marrow associated diseases such as AL amyloidosis, may comprise the steps:
(a) optionally administering an imaging RIC to the patient,
(b) administering a therapeutic RIC to the patient,
(c) optionally administering antibiotics, antimycotics and/or virustatics, and
(d) transplanting autologous or allogenic stem cells.

Antibiotics according to the present invention may be fluoroquinolone, ciprofloxacin (Cipro), levofloxacin (Levaquin/Quixin), gatifloxacin (Tequin), moxifloxacin (Avelox), ofloxacin (Ocuflox/Floxin/Floxacin), norfloxacin (Noroxin).

Virustatics according to the present invention may be aciclovir, ganciclovir, or valganciclovir.

Antimycotics used according to the present invention may be fluconazole, amphotericin B, Nystatin, Natamycin, Rimocidin, Filipin, Candicin, Hamycin, Perimycin, Dermostatin.

Preferred therapeutic protocols, particularly for the therapy of bone marrow associated diseases such AL-amyloidosis, comprise the steps of
(a) optionally administering an imaging RIC to a patient, particularly 2-10 days, more particularly 2-8 days, before step (b),
(b) administering a therapeutic RIC to the patient, particularly 6-16, more particularly 12-16 days, prior to step (c) and
(c) transplanting autologous or allogeneic stem cells.

A particularly preferred therapeutic protocol for the therapy of bone marrow associated diseases, particularly AL-amyloidosis, comprises the steps
(i) optionally administering an imaging RIC of the antibody BW 250/183 with ¹¹¹In to a patient, particularly 2-10 days, more particularly 2-8 days, before step (ii),
(ii) administering a therapeutic RIC of the antibody BW 250/183 with ⁹⁰Y to the patient, particularly 6-16 days, more particularly 12-16 days, prior to step (iii) and
(iii) transplanting autologous or allogeneic stem cells.

Surprisingly, it was found that for the treatment of bone marrow associated diseases such as AL-amyloidosis, no administration of an additional antitumor agent, such as melphalan or an immunosuppressive agent, is needed for conditioning bone marrow. Thus, treating such diseases with only a radioimmunoconjugate as conditioning agent minimizes or even avoids any side-effects typically associated with the standard conditioning with anti-tumor and/or immunosuppressive agents. The present invention allows treatment of transplant ineligible AL-amyloidosis patients.

It was further surprisingly found that patients suffering from bone marrow associated diseases such as AL-amyloidosis, who were treated with high-dose melphalan followed by stem cell transplantation, but had disease progression, could be treated with a therapeutic radioimmunoconjugate and subsequent stem cell transplantation according to the invention.

It was also found that patients suffering from bone marrow associated diseases such as AL-amyloidosis who were treated with induction therapy but had progression could be treated with a therapeutic radioimmunoconjugate and subsequent stem cell transplantation according to the invention.

The invention shall be explained in more detail by the following examples.

### Figures

- Figure 1: Preparation of the immunoconjugate CHX-A"-DTPA-anti-CD66 monoclonal antibody
- Figure 2: Flow diagram of the radiolabeling process for ¹¹¹Indium CHX-A"-DTPA-Anti CD66 drug product
- Figure 3: Flow Diagram of the radiolabeling process for ⁹⁰Yttrium CHX-A"-DTPA-Anti CD66 drug product
- Figure 4: Composition of ⁹⁰Yttrium labelled CHX-A"-DTPA-Anti CD66 drug product
- Figure 5: Sequential whole body gamma images following infusion of [¹¹¹In]-anti-CD66 drug product
- Figure 6a: Gamma camera images after [¹¹¹In]-anti-CD66 drug product infusion
- Figure 6b: Bremsstrahlung images after [⁹⁰Y]-anti-CD66 drug product infusion
- Figure 7: Patient's FLC response after ⁹⁰Y-anti-CD66 treatment

### Examples

### Material

### CD66-binding component

### Molecular Weight and Formula

The murine IgG1 kappa monoclonal antibody anti-CD66 BW 250/183 is monomeric with a molecular weight (MW) of 150 kDa protein and composed of IgG heavy and light chains of the expected MW (approx. 50 and 25 kDa, respectively). The antibody is also known as the below:
INN Name: Besilesomab
Chemical name: monoclonal antibody BW 250/183
Laboratory Code Name: MAb BW 250/183
CAS Registry Number: 537694-98-7

### Chelator

### NCS-CHX-A"-DTPA chelator

The synthetic bifunctional chelate used for the covalent conjugation event on the antibody is synthesized by Macrocyclics (1309 Record Crossing, Dallas, TX 75235, USA). The IUPAC name is 2,2'-(2-(((S)-1-(bis(carboxymethyl)amino)-3-(4-isothiocyanatophenyl)propan-2yl) (carboxymethyl)amino)propylazanediyl)diacetic acid, also known as isothiocyanato-(R)-2-amino-3-(phenyl)propyl)trans-(S,S)-cyclohexane-1,2-diamine-pentaacetic acid) (abbreviated to ITC-CHX-A"-DTPA or ITC-Bn-CHX-A"-DTPA). The low MW chelate (MW: 704 Da) has the following chemical formula C₂₆H₃₄N₄O₁₀S.3HCl and is presented as a solid off-white powder.

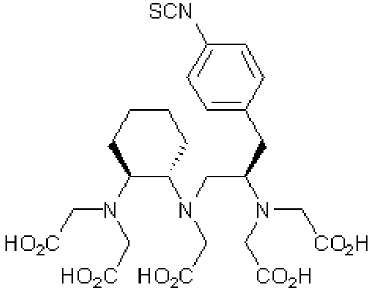

### Preparation of the radioimmunoconjugate

The batch size for the CHX-A"-DTPA-anti-CD66 Drug Substance Batch No. AB012 was 122 ml (355 mg), corresponding to 152 ml working volume (426 mg) of the starting monoclonal antibody preparation anti-CD66 Mab 250/183 (Orpegen Pharma GmbH or Glycotope Biotechnology GmbH, Germany or Celonic, Germany). The batch size for the CHX-A"-DTPA-anti-CD66 Drug Substance Batch No. AB013 was 250 ml (775 mg), corresponding to 376 ml working volume (1051 mg) of the starting monoclonal antibody preparation anti-CD66 Mab 250/183 (Orpegen Pharma GmbH).

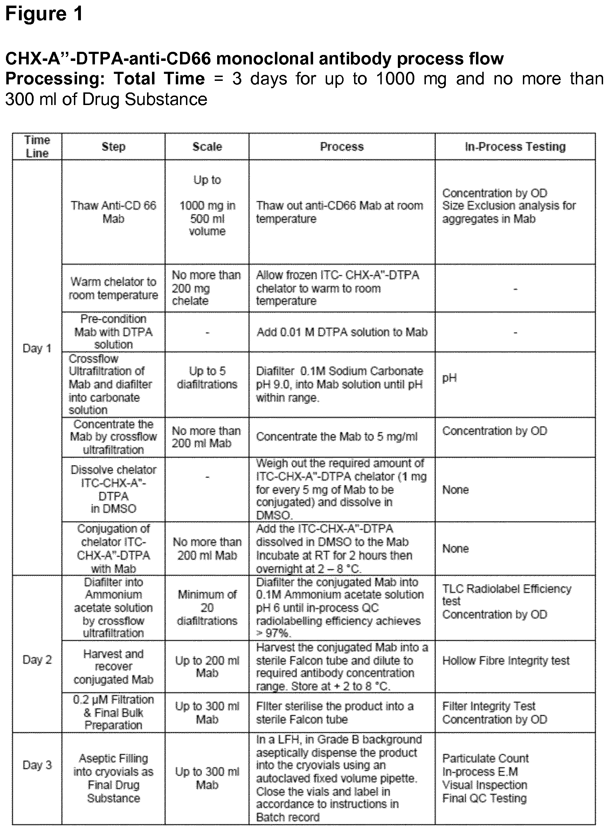

The manufacturing process and in-process controls conducted are outlined in Figure 1.

### a) Thawing out of materials

The first step in the process is the thawing out of the specific number of vials of anti-CD66 Mab specified in the batch record. The Mab is thawed at room temperature in a laminar flow hood (LFH) before combining the vials into a single sterile Falcon tube. An in-process QC sample is taken for size exclusion analysis to confirm that the Mab is predominantly monomeric and the results are reported.

The chelator ITC-CHX-A"-DTPA is removed from the freezer and allowed to warm to ambient temperature for a minimum of 1 hour at room temperature.

### b) Crossflow ultrafiltration of Mab - Diafiltration into 0.1M Sodium Carbonate pH 9.0 solution

The major processing step for the production of the Drug Substance process is the crossflow ultrafiltration/diafiltration (UF/DF) step. These cartridges are disposable, single use and available in a number of different molecular weight cut-offs for processing (Spectrum Laboratories, USA). The cartridge and UF/DF apparatus are pre-conditioned to remove glycerol as recommended by the manufacturer before sterilizing using a validated autoclaving cycle prior to use. In general, the UF/DF step is performed to filter, diafilter (buffer exchange) and concentrate the product throughout to predetermined specifications. The 50 kDa MW cut-off hollow fiber cartridge is operated within defined operating parameters (such as transmembrane pressures, velocity in a low shear manner) that allow the retention of the Mab, whilst allowing salts and other low molecular weight impurities to pass through. The cartridge and assembly is a closed process, which is operated within a grade A laminar flow hood in a grade B clean room background.

Before processing, a 1:10 vol:vol of 0.01M DTPA solution is added to the Mab solution in the LFH (i.e 1 ml of DTPA per 10 ml of Mab). The Mab is mixed gently and incubated at room temperature for 30 minutes. The Mab solution is introduced into the hollow fiber crossflow assembly in the LFH before the Mab is diafiltered with a minimum of 5 volumes of 0.1M Sodium Carbonate pH 9.0 until the pH is within the acceptable range. In-process QC samples are taken to confirm that the pH is within the 8.9-9.2 pH range required for conjugation. The Mab is recovered from the hollow fiber cartridge into a sterile Falcon tube. An in-process QC sample is taken to monitor the concentration of the Mab by OD 280 nm.

### c) Conjugation of Mab with chelate

The amount of ITC-CHX-A"-DTPA chelator required is calculated as follows - 1 mg for every 5 mg of Mab to be conjugated. The chelator is carefully weighed into a sterile container before dissolving in DMSO (dissolve 1 mg of chelator per 20 µl of DMSO). The ITC-CHX-A"-DTPA chelator is added carefully by steady mixing into the Mab (a small volume of carbonate buffer is used to wash out any remaining ligand from the container for adding to the Mab). The lid is replaced on the Mab container and the solution is mixed very gently by hand rotation. The tube is labelled and left at room temperature for 2 hours before incubation overnight at +2 to 8 °C.

### d) Crossflow ultrafiltration of Mab - Diafiltration/concentration into Acetate buffer

In an LFH, the Mab solution is diafiltered into 0.1M Ammonium acetate solution pH 6 until in-process QC radiolabeling efficiency achieves > 97%. Once the radiolabeling efficiency is > 97%, the Mab is recovered from the hollow fiber crossflow apparatus into a sterile Falcon tube by releasing the transmembrane pressure. An in-process QC sample is taken to determine the Mab concentration by OD 280 nm and, if required, the Mab is further diluted with the acetate buffer to within the acceptable range of 2.5-3.3 mg/ml. The final Mab solution is stored at +2 to 8 °C.

### e) Sterile filtration & filling of Drug Substance into cryovials

In an LFH, in a grade B background, the product is filter sterilized using a 0.22 µM filter into a sterile Falcon tube. The filter is integrity tested to ensure that it is within the acceptable bubble point range. In an LFH, in a grade B background, the product is aseptically dispensed into the sterile cryovials using an autoclaved fixed volume pipette. In processing, E.M is performed by the operators to include continuous air particle counts. The vials are closed and labelled in accordance to GMP before storage at less than -70 °C.

The bifunctional chelated CHX-A"-DTPA-anti-CD66 monoclonal antibody remains as a monomeric IgG1 of the expected MW size and composition.

The preparation is shown to maintain its specificity against the CD66 antigen by immunoreactivity assay.

CHX-A"-DTPA-anti-CD66 monoclonal antibody is readily soluble in water and appears as a colorless, clear solution with no visible particles when dissolved in ammonium acetate buffer pH 6.0.

### Drug product

### ¹¹¹Indium labelled CHX-A"-DTPA-Anti CD66 drug product (comparative)

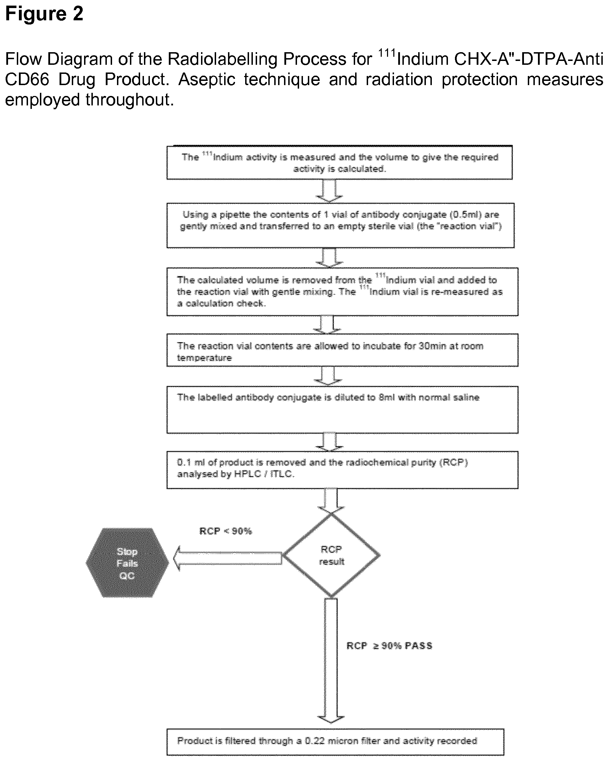

¹¹¹Indium labelled CHX-A"-DTPA-Anti CD66 drug product is manufactured in single dosage units for individual patients. The drug product ¹¹¹Indium labelled CHX-A"-DTPA-Anti CD66 will be prepared with an excess of ¹¹¹Indium to a maximum of 300 MBq to allow for residual activity losses during the manufacture and delay prior to infusion. A dose of 185 MBq ± 10% will be administered to each patient. The volume to be administered will be calculated based on the concentration of radioactivity in the vial. Residual radiolabeled product will be re-measured and reconciled against the original activity to allow accurate recording of actual activity administered to the patient. The flow diagram of the radiolabeling process for ¹¹¹Indium CHX-A"-DTPA-Anti CD66 drug product is shown in Figure 2.

¹¹¹Indium labelled CHX-A"-DTPA Anti-CD66 drug product is a monoclonal antibody conjugated to a bifunctional chelator, labelled with the radionuclide ¹¹¹Indium.

The two components of this radiolabeled antibody conjugate are:
1) CHX-A"-DTPA-Anti-CD66 Monoclonal antibody (the 'Drug Substance')
2) Radionuclide ¹¹¹Indium

The radiolabeling of the monoclonal antibody conjugate is prepared individually for each patient by the radiopharmacy departments at each site, following GMP guidelines, and is supplied as a sterile, pyrogen and particle-free solution for intravenous infusion containing besilesomab (1.0-1.5 mg) diluted to 8.0 ml with 0.9% sodium chloride solution BP, labelled with Indium-111 to give a final radioactive concentration of approximately 23 MBq/ml (acceptable range 20-30 MBq/ml) at the end of synthesis. The volume of each batch of drug product will be approximately 8-10 ml at the end of manufacture. The patient dose will be presented in one 10 mL syringe.

Alternatively, radiolabeling of the monoclonal antibody conjugate with In111 can be done centrally at a radiopharmaceutical manufacturing site and the Drug Product will be shipped at 4-8 °C to the respective transplantation center that treats the patient.

### ⁹⁰Yttrium labelled CHX-A"-DTPA-Anti CD66 drug product

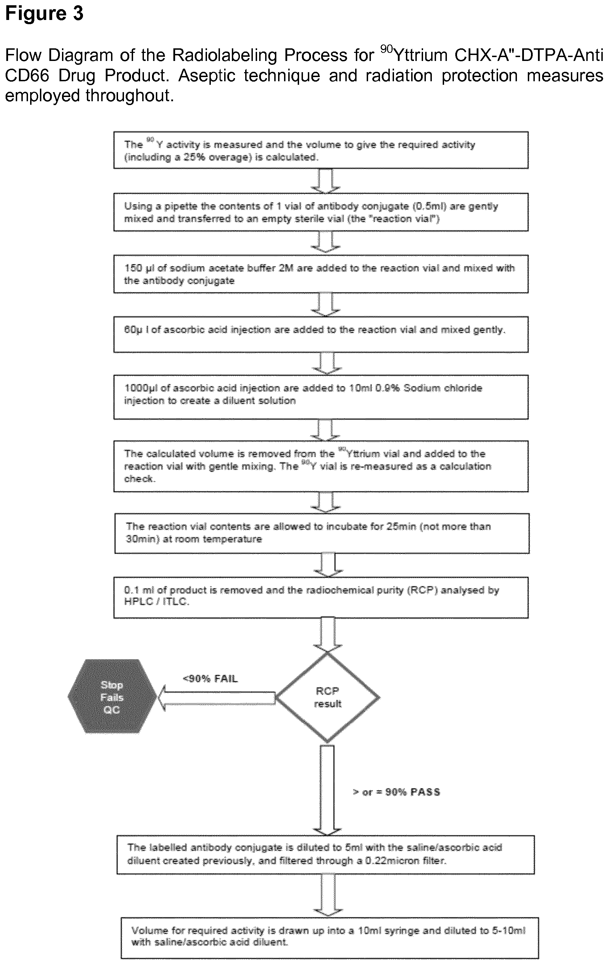

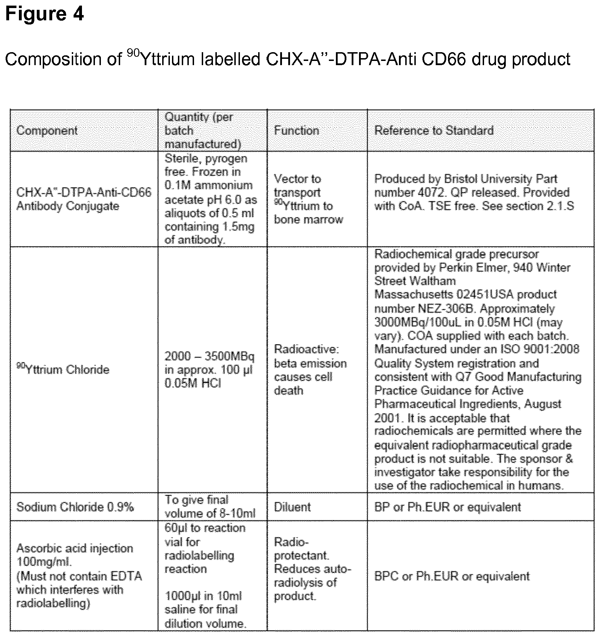

⁹⁰Yttrium labelled CHX-A"-DTPA-Anti CD66 drug product is manufactured in single dosage units for individual patients. The composition of each batch/single dosage unit is presented in Figure 4. The drug product ⁹⁰Yttrium CHX-A"-Anti-CD66 is prepared with an excess of approximately 25% ⁹⁰Yttrium to allow for residual activity losses during the manufacture. The actual dose administered to the patient will depend on the patient's lean body weight and the prescribed activity according to the protocol. The volume to be administered is calculated based on these factors together with the concentration of radioactivity in the vial. Residual radiolabeled product is re-measured and reconciled against the original activity to allow accurate recording of actual activity administered to the patient. The flow diagram of the radiolabeling process for ⁹⁰Yttrium CHX-A"-DTPA-Anti CD66 drug product is shown in Figure 3.

The radiolabeling of the monoclonal antibody conjugate is prepared individually for each patient by the radiopharmacy departments at each site, following GMP guidelines, and is supplied as a sterile, pyrogen and particle free solution for intravenous infusion containing CHX-A"-DTPA Anti-CD66 (1.0-1.5 mg) diluted to approximately 8.0 ml with 0.9% sodium chloride solution BP, labelled with ⁹⁰Yttrium to give a final radioactive concentration in the range of 200-500 MBq/ml at the end of synthesis.

Alternatively, radiolabeling of the monoclonal antibody conjugate with Y90 can be done centrally at a radiopharmaceutical manufacturing site and the Drug Product will be shipped at 4-8 °C to the respective transplantation center that treats the patient.

### Absorption properties (comparative)

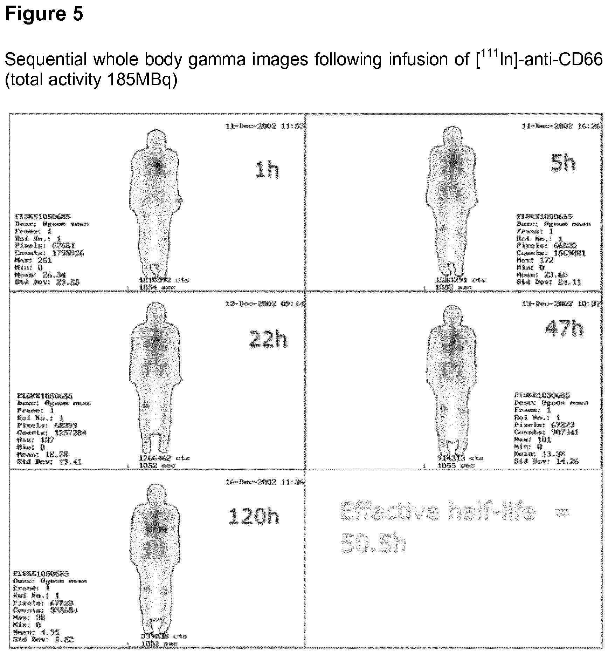

The radiolabeled ¹¹¹In anti-CD66 radioimmunoconjugate was administered intravenously in a buffered saline aqueous solution. Sequential gamma camera imaging demonstrates an initial blood phase and uptake by the bone marrow within 4-6 hours of infusion (Figure 5).

### Distribution

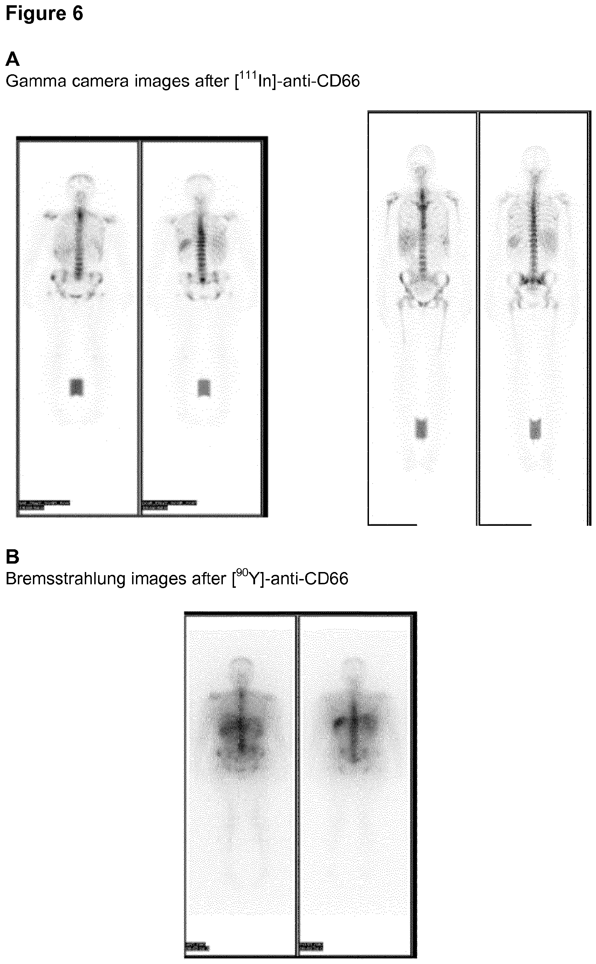

The biodistribution *in vivo* in humans shows consistent uptake by the cellular component of red marrow, typically in the axial skeleton (vertebrae, pelvis), ribs, skull and proximal region of long bones. Gamma images following infusion of comparative [¹¹¹In]-anti-CD66 are shown in Figure 6a and Bremsstrahlung images following infusion of [⁹⁰Y]-anti-CD66 are shown in Figure 6b, indicating similar biodistribution with each radiolabeled form of the anti-CD66.

### Objectives of the study

The main objective of the study is to determine the toxicity associated with the use of the [⁹⁰Y]-labelled anti-CD66 as the sole conditioning prior to autologous stem cell transplant in patients with AL-amyloidosis. Toxicity determination is measured by CTCAE version 4.0 criteria and stem cell engraftment and establishes the maximum tolerated radiation dose (MTD) over three infused radiation activity levels.

In addition, the study allows the assessment of clonal response (as measured by serum FLC assay) by using established validated methods of FLOW cytometry to measure the change in malignant plasma cell population. Disease response, cardiac recovery, time to progression and overall survival are also reviewed, whilst determining the engraftment status of patients.

Finally, the study allows the assessment of the dosimetry model previously developed in phase I and II trials in this patient group.

### Outcome measures/endpoints

Toxicity of [⁹⁰Y]-labelled anti-CD66 monoclonal antibody as the sole conditioning regime for autologous stem cell transplantation in patients with systemic AL-amyloidosis is determined using CTCAE version 4.0 criteria.

The clonal response using the [⁹⁰Y]-radiolabeled anti-CD66 mAb as targeted radiotherapy is measured by serial FLC assay. Responses will be summarized as recommended in the Consensus Guidelines for the conduct and reporting of clinical trials in systemic light-chain amyloidosis.

Clonal response to the [⁹⁰Y]-radiolabeled anti-CD66 mAb by following the change in malignant plasma cell population in bone marrow is determined using established validated methods of FLOW cytometry.

Disease response and cardiac recovery are determined by measuring NT-proBNP levels pre and post (D100) therapy.

Impact of using [⁹⁰Y]-labelled anti-CD66 mAb on time to progression and overall survival is assessed.

The utility of the dosimetry model developed in previous phase I and II trials using the same radiolabeled anti-CD66 and with imaging/dosimetry post ⁹⁰Y-labelled anti-CD66 is determined.

The engraftment of autologous stem cells is determined by time to platelet recovery > 50 x 10⁹/L and neutrophils > 0.5 x 10⁹/L (EBMT criteria).

The proportion of patients that form human anti-murine antibodies or human anti-mouse immunoglobulin antibodies (HAMA) following exposure to anti-CD66 mAb in the context of an autologous stem cell transplantation for AL amyloidosis is assessed.

### Trial design

This was an open label, phase I/IIa multi-center study, assessing the use of a radiolabeled anti-CD66 in patients with AL-amyloidosis with regard to
1) Safety and Toxicity
2) Disease response. Progression from one dose level to the next will be dependent on the toxicity profile demonstrated, but not based on disease response. Patients will be recruited from those attending the National Amyloidosis Centre for diagnosis and monitoring of their disease and in whom high-dose therapy would be a treatment option.

There are three treatment levels, representing increasing infused radiation activity levels:
1. 30.0 MBq/kg lean body weight [⁹⁰Y]-radio-labelled murine anti-CD66
2. 40.0 MBq/kg lean body weight [⁹⁰Y]-radio-labelled murine anti-CD66
3. 45.0 MBq/kg lean body weight [⁹⁰Y]-radio-labelled murine anti-CD66

### Toxicity

Patients will be recruited sequentially and receive treatment according to the dose level active at that time. As a safety factor, a gap equivalent to D+30 post-transplant must have passed before the next patient is treated at the same dose level and D+60 post-transplant between dose level changes (although patients may be recruited and consented before the previous patient reaches D+30 or D+60 post transplantation) assuming that no toxicities are recorded. If no patients show toxicity then a maximum of 3 patients will be recruited at that same dose level and the next dose level can start (after the interval equivalent to D+60). If 1 patient develops a defined toxicity, recruitment continues at the same dose level, but to a maximum of 6 patients at the same dose level. If 2 or more patients of the initial three patients treated in the same dose level develop toxicity, the study stops. If on review, the toxicities are determined by the independent data monitoring committee (IDMC) not to be directly due to the radiolabeled antibody, the trial can continue at the same dose level, but expanded to the maximum of 6 patients. If the IDMC determines that the toxicities are related directly to the radiolabeled antibody, the trial stops, the infused radiation activity level is then set as the MTD. After six patients, if more than 2 patients experience toxicities attributed by the IDMC to the radiolabeled antibody, the trial will stop, otherwise it can continue to the next dose level (as outlined above).

If, in the opinion of the IDMC, there have been no specific toxicities at any infused activity ('dose') level, the final infused activity level can be expanded to 6 patients to provide additional information on toxicity and disease response.

### Disease response

Disease response (change in free light chain (FLC)) will be determined in each patient over the study period. Because of the co-morbid conditions that can affect patients with AL-amyloidosis, it is not appropriate to demonstrate a clear MTD (i.e. by further increases in the infused radiation activity) if good clinical disease responses are achieved as indicated by reduction in the FLC and clonal plasma cell population post therapy and transplant. The optimal infused radiation would produce a CR rate in all patients without demonstrable toxicity. The results from the phase I study will be used to inform the format of further trials (phase II), which would be designed to determine the optimal infused radiation activity and hence estimated bone marrow radiation dose in this specific patient group. If no toxicity is demonstrated at any infused activity level, expansion of the third level to 6 patients will provide additional evidence for disease response.

### Patients

All patients with systemic AL-amyloidosis who meet eligibility criteria for ASCT in amyloidosis and have an indication for autologous stem cell transplantation as the preferred treatment option will be eligible for this study.

### Inclusion criteria

Patients with the following characteristics are eligible for this study
- aged ≥ 18 years
- diagnosis of systemic AL-amyloidosis, either as a new diagnosis or recurrent disease
- measurable clonal plasma cell dyscrasia
- amyloid-related organ dysfunction or organ syndrome
- estimated life expectancy of at least 6 months (as defined at trial entry)
- sufficient stem cells for two transplant procedures
- Bone Marrow (BM) cellularity > 20%
- eligible for ASCT in AL amyloidosis defined as fulfilling all of the following criteria:
   ∘ ECOG Performance Status of 0 or 1
   ∘ Cardiac troponin-T < 0.07 µg/L
   ∘ NYHA heart failure class of < 3
   ∘ no more than 3 organs involved by amyloidosis by consensus guidelines
   ∘ creatinine clearance or isotope GFR ≥ 30ml/min
   ∘ bilirubin ≤ 1.5 times and alkaline phosphatase ≤ 3 x upper limit of normal
   ∘ AST or ALT < 2.5 x upper limit of normal range
   ∘ mean left ventricular wall thicknesses of < 16mm by echocardiography
   ∘ absence of clinically important amyloid-related autonomic neuropathy
   ∘ absence of clinically important amyloid-related gastro-intestinal haemorrhage
- capable of providing written, informed consent
- women of child-bearing potential should use adequate forms of contraception
   ∘ Intrauterine Device (IUD)
   ∘ hormonal-based contraception (pill, contraceptive injection etc.)
   ∘ double barrier contraception (condom and occlusive cap e.g. diaphragm or cervical cap with spermicide)
   ∘ true abstinence (this is defined as refraining from heterosexual intercourse after receiving [¹¹¹In] at the Dosimetry and Imaging visit through to final study visit

### Exclusion criteria

Patients with the following characteristics are ineligible for this study:
- overt symptomatic multiple myeloma
- amyloidosis of unknown or non-AL type
- localized AL-amyloidosis (in which amyloid deposits are limited to a typical single organ, for example the bladder or larynx, in association with a clonal proliferative disorder within that organ)
- trivial or incidental AL amyloid deposits in the absence of a significant amyloid-related organ syndrome (e.g. isolated carpal tunnel syndrome).
- NYHA Class III or IV heart failure
- liver involvement by amyloid causing bilirubin > 1.5 times upper limit of normal
- concurrent active malignancies, except surgically removed basal cell carcinoma of the skin or other *in situ* carcinomas
- pregnant, lactating or unwilling to use adequate contraception
- intolerance/sensitivity to any of the study drugs
- known positive Human anti-murine antibodies or human anti-mouse immunoglobulin antibodies (HAMA)
- unable to provide written informed consent
- involved in another IMP trial

### Trial assessment

### Treatment - Visit 1

### Dosimetry and imaging

### Premedication of patients prior to administration of RIC

Patients receiving antibodies of animal origin occasionally experience symptoms, such as fever, chills, myalgia, even when lacking antibodies against the specific species, e.g. HAMA. Cumulative clinical experience with anti-CD66 indicates that side-effects due to the infusion of antibody are rare, reported as occurring in 0.5-1.0 per 10,000 infusions.

Resuscitation facilities must immediately be available; this includes apparatus for respiratory support - a supply of oxygen, ventilation, mask and bag. Adrenaline must be available and ready to be administered.

All subjects will receive pre-medication 30-60 minutes prior to the infusion of radiolabeled anti-CD66 consisting of 4 mg of chlorpheniramine and 1 g of paracetamol both orally, the use of steroids (e.g. hydrocortisone) will be avoided unless significant symptoms occur, in which case 50-100 mg of hydrocortisone will be given. If the use of radiolabeled anti-CD66 is associated with symptoms in the majority of subjects, the use of steroids as part of the premedication will be reviewed.

### Administration of [¹¹¹In]-labelled RIC and dosimetry (2-4 weeks after stem cell harvest) - drug product A

A small imaging dose of [¹¹¹In]-labelled anti-CD66, approximately 185 MBq, will be given.

### Treatment -Visit 2

### Administration of [⁹⁰Y]-labelled RIC (day -14)

Infusion of labelled anti-CD66 should take place at least a week after dosimetry and will be via a central venous line or, if one has not been inserted, a peripheral cannula with an initial infusion rate of 5 mg/hr for first 5 minutes; if tolerated, the rate may be increased to 10 mg/hr. The rate may need to be reduced or the infusion stopped if the subject experiences any significant symptoms, such as throat tightness, dyspnoea, severe chills or rigors. The line will be flushed with 10-20 ml of normal saline following completion of the infusion.

At these rates of infusion, the total of 2 mg of labelled antibody will take 12 minutes to infuse.

### Treatment - Visit 3 (day -7 to day 0 post [⁹⁰Y])

Patients will be reviewed and commenced on prophylactic antibiotics (Ciprofloxacin 250 mg bd, Aciclovir 400 mg bd and Fluconazole 100 mg od).

### Autologous Stem Cell Reinfusion (Day 0)

All patients will be transplanted at the transplant centers using either inpatient or ambulatory care facilities as appropriate. Based on the experience of the phase I and II trials using the same [⁹⁰Y]-anti-CD66, patient's peripheral blood counts are anticipated to fall from D7 post [⁹⁰Y]-infusion reaching a nadir at D8-10 [equivalent to D-6 to D-4 pre-transplant]. Patients will then be admitted if not already treated as in-patients. Following their stem cell transplant (Day 0), they will be assessed daily by the study team for adverse events and appropriate need for additional antibiotics or blood products. Patients will be discharged from the hospital after adequate haematopoietic engraftment and recovery from any infections and if independent of blood product support. The patient will be followed up after discharge at day 30 and day 100. Trial follow-up will finish at day 100 and follow-up will continue off trial as per standard medical practice.

Time to disease recurrence or progression and time to next treatment will not be included as part of the formal assessment within the context of the trial. However, all patients undergoing ASCT for AL-amyloidosis are routinely on long-term follow-up by their referring consultant and are also regularly reviewed.

### Results

The first three AL-amyloidosis patients that had been treated with high-dose melphalan and subsequent ASCT and had a relapse were treated with 30 MBq/kg of ⁹⁰Y-labelled drug product after appropriate dosimetry with ¹¹¹In-labelled drug product.

The three patients did not show any side-effects during conditioning with ⁹⁰Y-labelled drug product, no Severe Adverse Reactions (SAES) or Suspected Unexpected Severe Adverse Reactions (SUSARs) were seen. In all three patients, a fall in clonal free light chain was observed. In one case a complete response with sustained normalization of FLC and improvement in clinical symptoms and disease parameters of AL-amyloidosis was seen. The trial has been given approval by the Data and Safety Committee to continue to the next infused activity level.

These findings are absolutely unique and unexpected. Induction of disease regression as indicated by the FLC reduction without any side-effects is an outstanding result, which delivers major benefit to patients suffering from AL-amyloidosis.

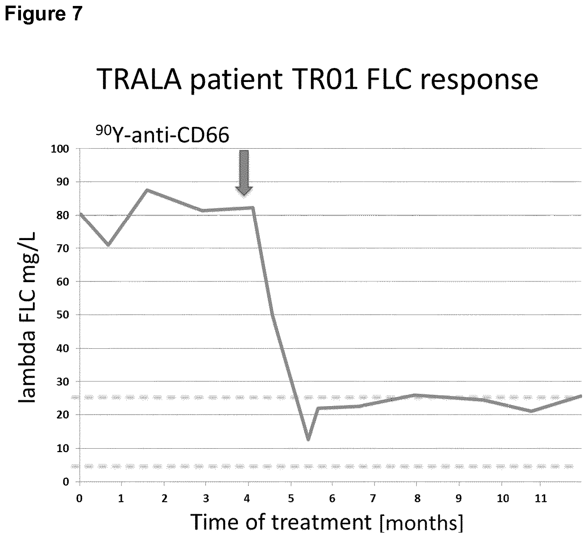

Figure 7 shows the concentration of serum free light chain in a patient subjected to RIC treatment at a dose of 30 MBq/kg over time. The FLC concentration, which is a reliable biomarker of AL-amyloidosis, decreases from 80 mg/l to about 25 mg/l within one month. The FLC level is maintained at between 10 and 30 mg/l - which is a typical value in a healthy human - for several months. One patient had a complete remission, which was analyzed by normalization of free light chains, bone marrow biopsy and determination of the minimal residual disease. The patient has been in biochemical complete remission for 15 months following transplant.

The two other patients also showed a significant decrease of the FLC level after RIC treatment.

## Claims

1. Radioimmunoconjugate (RIC) for use as a medicament in bone-marrow conditioning, particularly preceding stem cell transplantation, wherein the RIC comprises a CD66-binding component and a therapeutically effective radionuclide,
wherein the CD66-binding component is an antibody,
wherein the radionuclide is linked to the CD66-binding component via a chelating agent, which is covalently linked to the CD66-binding component and
wherein the therapeutic RIC is for administration to a patient who is ineligible to treatment with high dose-melphalan preceding HSCT and/or to a patient who was treated with induction therapy but had disease progression.

2. Radioimmunoconjugate (RIC) for use in treating bone marrow-associated diseases namely AL-amyloidosis, wherein the RIC comprises a CD66-binding component and a therapeutically effective radionuclide, wherein the CD66-binding component is an antibody and wherein the radionuclide is linked to the CD66-binding component via a chelating agent, which is covalently linked to the CD66-binding component.

3. Radioimmunoconjugate (RIC) for the use according to claim 1 or 2, wherein the radionuclide is yttrium-90 (⁹⁰Y).

4. Radioimmunoconjugate (RIC) for the use according to any of claims 1-3, wherein the CD66-binding component is the antibody BW 250/183.

5. Radioimmunoconjugate (RIC) for the use according to claims 1-4, wherein the radionuclide is linked to the CD66-binding component via a structure of the formula
[(chelating agent)-(R¹)ₚ-(R²-R³)ₙ]ₘ-(CD66-binding component)
wherein n is 0 or 1,
m is 1 to 15,
p is 0 or 1,
R¹ and R³ are independently selected from the group consisting of - NHCSNH-, -NHCONH-, -NHCOCH₂S-, -S-S-, -NH-NH-, -NH-, -S-, - CONHNH-, -SCH₂CH₂COONH-, -SCH₂CH₂SO₂-, -SCH₂CH₂SO₂NH-, -CONH-, -O-CH₂CH₂O-, -CO-, -COO-, -NH-O-, -CONHO-, -S-(CH₂)₃C(NH)NH-, -NH-COO-, -O- and 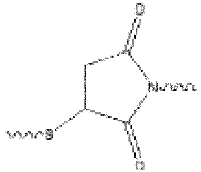
preferably -NH-CS-NH-, and
R² is selected from the group consisting of C1-C18 alkylen, branched C1-C18, -CH₂-C₆H₁₀-, *p*-alkylphenylene, *p*-phenylene, *m*-phenylene, p-alkyloxyphenylene, naphthylene, -[CH₂CH₂O]ₓ-, -[CH₂CH₂SOCH₂CH₂]ₓ-, - [CH₂CH₂SO₂CH₂CH₂]ₓ-, or -[NHCHR₄CO]_{y}-, wherein x is 1 to 200, y is 1 to 20, and wherein R₄ is selected from the group consisting of H-, Me-, HSCH₂-, isopropyl, but-2-yl, CH₃SCH₂CH₂-, benzyl, 1*H*-indol-3-yl-methyl, HOCH₂-, HOOCCH₂-, CH₃CH(OH)-, HOOCCH₂CH₂-, 4-hydroxybenzyl, H₂NCOCH₂-, H₂NCOCH₂CH₂-, 4-aminobut-1-yl, 2-guanidinoethyl, 1*H*-imidazol-5-yl-methyl and 2-methylprop-1-yl.

6. Radioimmunoconjugate (RIC) for the use according to any of claims 4-5, wherein the chelating agent is selected from the group consisting of diethylenetriaminepentaacetic acid (DTPA), 1,4,7,10-tetraazacyclododecane-N,N',N",N‴-tetraacetic acid (DOTA), 1, 4, 8,11-tetraazacyclotetradecane-N,N',N",N‴-tetraacetic acid (TETA), 1,4,7-triazonane-N,N',N"-triacetic acid (NOTA), 2,2'-(2-(((1S,2S)-2-(bis(carboxymethyl)amino)cyclohexyl)-(carboxymethyl)amino)ethylazanediyl)diacetic acid (cyclohexano-DTPA), 2,2'-(2-(((1R,2R)-2-(bis(carboxymethyl)amino)cyclohexyl)-(carboxymethyl)amino)ethylazanediyl)diacetic acid, 2,2'-(2-(((1S,2R)-2-(bis(carboxymethyl)amino)cyclohexyl)-(carboxymethyl)amino)ethylazanediyl)diacetic acid, 2,2'-(2-(((1R,2S)-2-(bis(carboxymethyl)amino)cyclohexyl)-(carboxymethyl)amino)ethylazanediyl)diacetic acid, 2,2',2",2‴-(2,2'-(1S,2S)-cyclohexane-1,2-diylbis((carboxymethyl)azanediyl)bis(ethane-2,1-diyl))bis(azanetriyl)tetraacetic acid, 2,2',2",2‴-(2,2'-(1S,2R)-cyclohexane-1,2-diylbis((carboxymethyl)azanediyl)bis(ethane-2,1-diyl))bis(azanetriyl)tetraacetic acid, (1*R*)-1-benzyl-diethylenetriaminepentaacetic acid, (1*S*)-1-benzyl-diethylenetriaminepentaacetic acid, (2*R*)-2-benzyl-diethylenetriaminepentaacetic acid, (2*S*)-2-benzyl-diethylenetriaminepentaacetic acid, (2*R*)-2-benzyl-(3*R*)-3-methyl-DTPA, (*2*R)-2-benzyl-(3*S*)-3-methyl-DTPA, (2*S*)-2-benzyl-(3*S*)-3-methyl-DTPA, (2*S*)-2-benzyl-(3*R*)-3-methyl-DTPA, (2*R*)-2-benzyl-(4*R*)-4-methyl-DTPA, (2*R*)-2-benzyl-(4*S*)-4-methyl-DTPA, (2*S*)-2-benzyl-(4*S*)-4-methyl-DTPA, (2*S*)-2-benzyl-(4*R*)-4-methyl-DTPA, (1*R*)-1-benzyl-(3*R*)-3-methyl-DTPA, (1*R*)-1-benzyl-(3*S*)-3-methyl-DTPA, (1*S*)-1-benzyl-(3*S*)-3-methyl-DTPA, (1*S*)-1-benzyl-(3*R*)-3-methyl-DTPA, (1*R*)-1-benzyl-(4*R*)-4-methyl-DTPA, (1*R*)-1-benzyl-(4*S*)-4-methyl-DTPA, (1*S*)-1-benzyl-(4*S*)-4-methyl-DTPA, (1*S*)-1-benzyl-(4*R*)-4-methyl-DTPA, 2,2'-((1R,2R)-2-(((R)-2-(bis(carboxymethyl)amino)-3-phenylpropyl)(carboxymethyl)amino)cyclohexylazanediyl)diacetic acid, 2,2'-((1S,2S)-2-(((S)-2-(bis(carboxymethyl)amino)-3-phenylpropyl)(carboxymethyl)amino)cyclohexylazanediyl)diacetic acid, 2,2'-((1R,2R)-2-(((S)-2-(bis(carboxymethyl)amino)-3-phenylpropyl)(carboxymethyl)amino)cyclohexylazanediyl)diacetic acid, 2,2'-((1S,2S)-2-(((R)-2-(bis(carboxymethyl)amino)-3-phenylpropyl)(carboxymethyl)amino)cyclohexylazanediyl)diacetic acid, 2,2'-((1R,2S)-2-(((R)-2-(bis(carboxymethyl)amino)-3-phenylpropyl)(carboxymethyl)amino)cyclohexylazanediyl)diacetic acid, 2,2'-((1S,2R)-2-(((S)-2-(bis(carboxymethyl)amino)-3-phenylpropyl)(carboxymethyl)amino)cyclohexylazanediyl)diacetic acid, 2,2'-((1S,2R)-2-(((R)-2-(bis(carboxymethyl)amino)-3-phenylpropyl)(carboxymethyl)amino)cyclohexylazanediyl)diacetic acid, 2,2'-((1R,2S)-2-(((S)-2-(bis(carboxymethyl)amino)-3-phenylpropyl)(carboxymethyl)amino)cyclohexylazanediyl)diacetic acid, (2*S*)-2-benzyl-1,4,7,10-tetraazacyclododecane-N,N',N",N‴-tetraacetic acid, (2*R*)-2-benzyl-1 ,4,7,1 0-tetraazacyclododecane-N, N',N ", N‴-tetraacetic acid, 6-benzyl-1,4,8,11-tetraazacyclotetradecane-N,N',N",N‴-tetraacetic acid, 2-benzyl-1 ,4,7-triazonane-N,N',N"-triacetic acid, benzyl-3-methyl-diethylenetriaminepentaacetic acid (2B3M-DTPA), (R)-2-amino-3-(phenyl)propyl)trans-(S,S)-cyclohexane-1,2-diamine-pentaacetic acid) (Bn-CHX-A"-DTPA) and salts and derivatives thereof,
particularly (R)-2-amino-3-(phenyl)propyl)trans-(S,S)-cyclohexane-1,2-diamine-pentaacetic acid) (Bn-CHX-A"-DTPA) and salts thereof.

7. Radioimmunoconjugate (RIC) for the use according to any of claims 1-6, wherein the radionuclide is linked to the CD66-binding component via the structure CD66-binding component-NH-CS-NH-Bn-CHX-A"-DTPA.

8. Radioimmunoconjugate (RIC) for the use according to any of claims 1-7, wherein a therapeutic RIC is administered at a dose of ≥ about 10 MBq/kg lean body weight (!bw), preferably ≥ about 15 MBq/kg lbw, more preferably ≥ about 20 MBq/kg lbw, still more preferably ≥ about 25 MBq/kg lbw, still more preferably ≥ about 30 MBq/kg lbw and still more preferably ≥ about 35 MBq/kg lbw, up to 60 MBq/kg lbw, preferably up to 45 MBq/kg lbw.

9. Radioimmunoconjugate (RIC) for the use according to any of claims 2-8, wherein
treatment of bone marrow associated diseases namely AL-amyloidosis further comprises stem cell transplantation, particularly autologous or allogeneic stem cell transplantation, more particularly autologous stem cell transplantation, particularly wherein
the therapeutic RIC is for administration 6-16 days, preferably 6-12 days, before stem cell transplantation and/or
wherein treatment of bone marrow associated diseases such as AL-amyloidosis does not involve administration of an additional anti-tumor agent, such as melphalan, or an immunosuppressive agent at least four weeks before and/or after stem cell transplantation.

10. Radioimmunoconjugate (RIC) for the use according to any of claims 2-10, wherein the
therapeutic RIC is for administration to a patient who was treated with high-dose melphalan followed by stem cell transplantation but had disease progression and/or
is for administration to a patient who was treated with induction therapy but had disease progression.

11. Radioimmunoconjugate (RIC) for the use according to any of claims 2-11, wherein the therapeutic RIC is for administration to a patient who is ineligible to treatment with HD-melphalan preceding HSCT.

12. Radioimmunoconjugate (RIC) for the use according to any one of claims 2-11, wherein treatment of bone marrow associated diseases namely AL-amyloidosis comprises the steps of
(a) optionally administering an imaging RIC to a patient, particularly 2-10 days before step (b), wherein the imaging RIC particularly comprises an indium-111 (¹¹¹In) radionuclide,
(b) administering a therapeutic RIC to the patient, particularly 6-16 days prior to step (c), and
(c) transplanting autologous or allogeneic stem cells.

13. Radioimmunoconjugate (RIC) for the use according to claims 1, 3, 4-8 and 11, wherein the stem cell transplantation is autologous or allogeneic.

## Patentansprüche

1. Radioimmunkonjugat (RIC) zur Verwendung als Medikament bei der Konditionierung von Knochenmark, insbesondere vor einer Stammzelltransplantation, wobei das RIC eine CD66-bindende Komponente und ein therapeutisch wirksames Radionuklid umfasst,
wobei die CD66-bindende Komponente ein Antikörper ist,
wobei das Radionuklid über einen Chelatbildner, der kovalent an die CD66-bindende Komponente gebunden ist, an der CD66-bindenden Komponente gebunden ist und
wobei das therapeutische RIC zur Verabreichung an einen Patienten ist, der für eine Behandlung mit hochdosiertem Melphalan vor einer HSCT nicht in Frage kommt und/oder an einen Patienten, der mit einer Induktionstherapie behandelt wurde, aber eine Krankheitsprogression zeigte.

2. Radioimmunkonjugat (RIC) zur Verwendung bei der Behandlung von Erkrankungen, die mit dem Knochenmark verbunden sind, nämlich AL-Amyloidose, wobei das RIC eine CD66-bindende Komponente und ein therapeutisch wirksames Radionuklid umfasst, wobei die CD66-bindende Komponente ein Antikörper ist und wobei das Radionuklid über einen Chelatbildner, der kovalent an die CD66-bindende Komponente gebunden ist, an der CD66-bindenden Komponente gebunden ist.

3. Radioimmunkonjugat (RIC) zur Verwendung nach Anspruch 1 oder 2, wobei das Radionuklid Yttrium-90 (⁹⁰Y) ist.

4. Radioimmunkonjugat (RIC) zur Verwendung nach einem der Ansprüche 1-3, wobei die CD66-bindende Komponente der Antikörper BW 250/183 ist.

5. Radioimmunkonjugat (RIC) zur Verwendung nach den Ansprüchen 1-4, wobei das Radionuklid an der CD66-bindenden Komponente über eine Struktur der Formel
[(Chelatbildner)-(R₁)ₚ-(R²-R³)ₙ]ₘ-(CD66-bindende Komponente)
gebunden ist,
wobei n 0 oder 1 ist,
m 1 bis 15 ist,
p 0 oder 1 ist,
R¹ und R³ unabhängig voneinander ausgewählt sind aus der Gruppe bestehend aus -NHCSNH-, - NHCONH-, -NHCOCH₂S-, -S-S-, -NH-NH-, -NH-, -S-, -CONHNH-, -SCH₂CH₂COONH-, -SCH₂CH₂SO₂-, - SCH₂CH₂SO₂NH-, -CONH-, -O-CH₂CH₂O-, -CO-, -COO-, -NH-O-, -CONHO-, -S-(CH₂)₃C(NH)NH-, -NH-COO-, -O- und 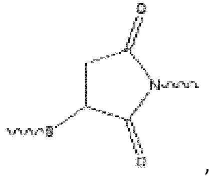
bevorzugt -NH-CS-NH-, und
R² ausgewählt ist aus der Gruppe bestehend aus C1-C18-Alkylen, verzweigtem C1-C18, -CH₂-C₆H₁₀-, p-Alkylphenylen, *p*-Phenylen, *m*-Phenylen, *p*-Alkyloxyphenylen, Naphthylen, -[CH₂CH₂O]ₓ-, - [CH₂CH₂SOCH₂CH₂]ₓ-, -[CH₂CH₂SO₂CH₂CH₂]ₓ-, oder -[NHCHR₄CO]_{y}-, wobei x 1 bis 200 ist, y 1 bis 20 ist und wobei R₄ ausgewählt ist aus der Gruppe bestehend aus H-, Me-, HSCH₂-, Isopropyl, But-2-yl, CH₃SCH₂CH₂-, Benzyl, 1*H*-Indol-3-yl-methyl, HOCH₂-, HOOCCH₂-, CH₃CH(OH)-, HOOCCH₂CH₂-, 4-Hydroxybenzyl, H₂NCOCH₂-, H₂NCOCH₂CH₂-, 4-Aminobut-1-yl, 2-Guanidinoethyl, 1*H*-Imidazol-5-yl-methyl und 2-Methylprop-1-yl.

6. Radioimmunkonjugat (RIC) zur Verwendung nach einem der Ansprüche 4-5, wobei der Chelatbildner ausgewählt ist aus der Gruppe bestehend aus Diethylentriaminpentaessigsäure (DTPA), 1,4,7,10-Tetraazacyclododecan-N,N',N",N‴-Tetraessigsäure (DOTA), 1,4,8,11-Tetraazacyclotetradecan-N,N',N",N‴-Tetraessigsäure (TETA), 1,4,7-Triazonan-N,N',N"-Triessigsäure (NOTA), 2,2'-(2-(((1S,2S)-2-(Bis(carboxymethyl)amino)cyclohexyl)-(carboxymethyl)amino)ethylazandiyl)diessigsäure (Cyclohexan-DTPA), 2,2'-(2-(((1R,2R)-2-(Bis(carboxymethyl)amino)cyclohexyl)-(carboxymethyl)amino)ethylazandiyl)diesessigsäure, 2,2'-(2-(((1S,2R)-2-(Bis(carboxymethyl)amino)cyclohexyl)-(carboxymethyl)amino)ethylazandiyl)diessigsäure, 2,2'-(2-(((1R,2S)-2-(Bis(carboxymethyl)amino)cyclohexyl)-(carboxymethyl)amino)ethylazandiyl)diessigsäure, 2,2',2",2"'-(2,2'-(1S,2S)-cyclohexan-1,2-diylbis((carboxymethyl)azandiyl)bis(ethan-2,1-diyl))bis(azantriyl)tetraessigsäure, 2,2',2",2"-(2,2'-(1S,2R)-Cyclohexan-1,2-diylbis((carboxymethyl)azandiyl)bis(ethan-2,1)-diyl))bis(azantriyl)tetraessigsäure, (1*R*)-1-Benzyl-diethylentriaminpentaessigsäure, (1*S*)-1-Benzyl-diethylentriaminpentaessigsäure, (2*R*)-2-Benzyl-diethylentriaminpentaessigsäure, (2*S*)-2-Benzyl-diethylentriaminpentaessigsäure, (2*R*)-2-Benzyl-(3*R*)-3-methyl-DTPA, (*2*R)-2-Benzyl-(3*S*)-3-methyl-DTPA, (2*S*)-2-Benzyl-(3*S*)-3-methyl-DTPA, (2*S*)-2-Benzyl-(3*R*)-3-methyl-DTPA, (2*R*)-2-Benzyl-(4*R*)-4-methyl-DTPA, (2*R*)-2-Benzyl-(4*S*)-4-methyl-DTPA, (2*S*)-2-Benzyl-(4*S*)-4-methyl-DTPA, (2*S*)-2-Benzyl-(4*R*)-4-methyl-DTPA, (1*R*)-1-Benzyl-(3*R*)-3-methyl-DTPA, (1*R*)-1-Benzyl-(3*S*)-3-methyl-DTPA, (1*S*)-1-Benzyl-(3*S*)-3-methyl-DTPA, (1*S*)-1-Benzyl-(3*R*)-3-methyl-DTPA, (1*R*)-1-Benzyl-(4*R*)-4-methyl-DTPA, (1*R*)-1-Benzyl-(4*S*)-4-methyl-DTPA, (1*S*)-1-Benzyl-(4*S*)-4-methyl-DTPA, (1*S*)-1-Benzyl-(4*R*)-4-methyl-DTPA, 2,2'-((1R,2R)-2-(((R)-2-(Bis(carboxymethyl)amino)-3-phenylpropyl)(carboxymethyl)amino)cyclohexylazandiyl)diessigsäure, 2,2'-((1S,2S)-2-(((S)-2-(Bis(carboxymethyl)amino)-3-phenylpropyl)(carboxymethyl)amino)cyclohexylazandiyl)diessigsäure, 2,2'-((1R,2R)-2-(((S)-2-(Bis(carboxymethyl)amino)-3-phenylpropyl)(carboxymethyl)amino)cyclohexylazandiyl)diessigsäure, 2,2'-((1S,2S)-2-(((R)-2-(Bis(carboxymethyl)amino)-3-phenylpropyl)(carboxymethyl)amino)cyclohexylazandiyl)diessigsäure, 2,2'-((1R,2S)-2-(((R)-2-(Bis(carboxymethyl)amino)-3-phenylpropyl)(carboxymethyl)amino)cyclohexylazandiyl)diessigsäure, 2,2'-((1S,2R)-2-(((S)-2-(Bis(carboxymethyl)amino)-3-phenylpropyl)(carboxymethyl)amino)cyclohexylazandiyl)diessigsäure, 2,2'-((1S,2R)-2-(((R)-2-(Bis(carboxymethyl)amino)-3-phenylpropyl)(carboxymethyl)amino)cyclohexylazandiyl)diessigsäure, 2,2'-((1R,2S)-2-(((S)-2-(Bis(carboxymethyl)amino)-3-phenylpropyl)(carboxymethyl)amino)cyclohexylazandiyl)diessigsäure, (2S)-2-Benzyl-1,4,7,10-tetraazacyclododecan-N,N',N",N"'-tetraessigsäure, (2*R*)-2-Benzyl-1,4,7,10-tetraazacyclododecan-N,N',N",N‴-tetraessigsäure, 6-Benzyl-1,4,8,11-tetraazacyclotetradecan-N,N',N",N‴-tetraessigsäure, 2-Benzyl-1,4,7-triazonan-N,N',N"-triessigsäure, Benzyl-3-methyl-diethylentriaminpentaessigsäure (2B3M-DTPA), (R)-2-Amino-3-(phenyl)propyl)trans-(S,S)-cyclohexan-1,2-diamin-pentaessigsäure) (Bn-CHX-A"-DTPA) und Salzen und Derivaten davon, insbesondere (R)-2-Amino-3-(phenyl)propyl)trans-(S,S)-cyclohexan-1,2-diamin-pentaessigsäure) (Bn-CHX-A"-DTPA) und Salzen davon.

7. Radioimmunkonjugat (RIC) zur Verwendung nach einem der Ansprüche 1-6, wobei das Radionuklid über die Struktur CD66-bindende Komponente-NH-CS-NH-Bn-CHX-A"-DTPA an die CD66-bindende Komponente gebunden ist.

8. Radioimmunkonjugat (RIC) zur Verwendung nach einem der Ansprüche 1-7, wobei ein therapeutisches RIC in einer Dosis von ≥ etwa 10 MBq/kg magerem Körpergewicht (Ibw), vorzugsweise ≥ etwa 15 MBq/kg Ibw, stärker bevorzugt ≥ etwa 20 MBq/kg Ibw, noch bevorzugter ≥ etwa 25 MBq/kg Ibw, noch bevorzugter ≥ etwa 30 MBq/kg Ibw und noch bevorzugter ≥ etwa 35 MBq/kg Ibw, bis zu 60 MBq/kg Ibw, vorzugsweise bis zu 45 MBq/kg Ibw verabreicht wird.

9. Radioimmunkonjugat (RIC) zur Verwendung nach einem der Ansprüche 2-8, wobei die Behandlung von Erkrankungen, die mit dem Knochenmark verbundenen sind, nämlich AL-Amyloidose, ferner eine Stammzelltransplantation, insbesondere eine autologe oder allogene Stammzelltransplantation, insbesondere eine autologe Stammzelltransplantation, umfasst, insbesondere wobei
das therapeutische RIC zur Verabreichung 6-16 Tage, vorzugsweise 6-12 Tage, vor der Stammzelltransplantation vorgesehen ist und/oder
wobei die Behandlung von Erkrankungen, die mit dem Knochenmark verbundenen sind, wie AL-Amyloidose, keine Verabreichung eines zusätzlichen Antitumormittels wie Melphalan oder eines Immunsuppressivums mindestens vier Wochen vor und/oder nach einer Stammzelltransplantation umfasst.

10. Radioimmunkonjugat (RIC) zur Verwendung nach einem der Ansprüche 2-10, wobei das therapeutische RIC zur Verabreichung an einen Patienten bestimmt ist, der mit hochdosiertem Melphalan gefolgt von Stammzelltransplantation behandelt wurde, jedoch eine Krankheitsprogression aufwies und/oder
zur Verabreichung an einen Patienten bestimmt ist, der mit einer Induktionstherapie behandelt wurde, jedoch eine Krankheitsprogression aufwies.

11. Radioimmunkonjugat (RIC) zur Verwendung nach einem der Ansprüche 2-11, wobei das therapeutische RIC zur Verabreichung an einen Patienten bestimmt ist, der für eine Behandlung mit HD-Melphalan vor einer HSCT nicht in Frage kommt.

12. Radioimmunkonjugat (RIC) zur Verwendung nach einem der Ansprüche 2-11, wobei die Behandlung von Erkrankungen, die mit dem Knochenmark verbundenen sind, nämlich AL-Amyloidose, die Schritte umfasst:
(a) optionales Verabreichen eines bildgebenden RIC an einen Patienten, insbesondere 2-10 Tage vor Schritt (b), wobei das bildgebende RIC insbesondere ein Indium-111 (¹¹¹In) Radionuklid umfasst,
(b) Verabreichen eines therapeutischen RIC an den Patienten, insbesondere 6-16 Tage vor Schritt (c), und
(c) Transplantation autologer oder allogener Stammzellen.

13. Radioimmunkonjugat (RIC) zur Verwendung nach den Ansprüchen 1, 3, 4-8 und 11, wobei die Stammzelltransplantation autolog oder allogen ist.

## Revendications

1. Radioimmunoconjugué (RIC) pour son utilisation en tant que médicament dans le conditionnement de moelle osseuse, notamment avant une transplantation de cellules souches, dans lequel le RIC comprend un composant de liaison à CD66 et un radionucléide thérapeutiquement efficace,
dans lequel le composant de liaison à CD66 est un anticorps,
dans lequel le radionucléide est lié au composant de liaison à CD66 via un agent chélatant, qui est lié de manière covalente au composant de liaison à CD66 et
dans lequel le RIC thérapeutique est destiné à être administré à un patient qui n'est pas éligible à un traitement avec du melphalan à haute dose avant une HSCT et/ou à un patient qui a été traité avec une thérapie d'induction mais dont la maladie a progressé.

2. Radioimmunoconjugué (RIC) pour son utilisation dans le traitement de maladies associées à la moelle osseuse, précisément l'AL-amyloïdose, dans lequel le RIC comprend un composant de liaison à CD66 et un radionucléide thérapeutiquement efficace, dans lequel le composant de liaison à CD66 est un anticorps et dans lequel le radionucléide est lié au composant de liaison à CD66 via un agent chélatant, qui est lié de manière covalente au composant de liaison à CD66.

3. Radioimmunoconjugué (RIC) pour son utilisation selon la revendication 1 ou 2, dans lequel le radionucléide est l'yttrium-90 (⁹⁰Y).

4. Radioimmunoconjugué (RIC) pour son utilisation selon l'une quelconque des revendications 1 à 3, dans lequel le composant de liaison à CD66 est l'anticorps BW 250/183.

5. Radioimmunoconjugué (RIC) pour son utilisation selon l'une quelconque des revendications 1 à 4, dans lequel le radionucléide est lié au composant de liaison à CD66 via une structure de formule
[(agent chélatant)-(R¹)ₚ-(R²-R³)ₙ]ₘ-(composant de liaison à CD66)
dans laquelle n est 0 ou 1,
m est 1 à 15,
p est 0 ou 1,
R¹ et R³ sont indépendamment sélectionnés dans le groupe constitué de -NHCSNH-, -NHCONH-, -NHCOCH₂S-, -S-S-, -NH-NH-, -NH-, -S-, -CONHNH-, - SCH₂CH₂COONH-, -SCH₂CH₂SO₂-, -SCH₂CH₂SO₂NH-, -CONH-, -O-CH₂CH₂O-, - CO-, -COO-, -NH-O-, -CONHO-, -S-(CH₂)₃C(NH)NH-, -NH-COO-, -O- et 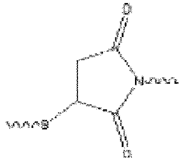
de préférence -NH-CS-NH-, et
R² est sélectionné dans le groupe constitué d'alkylène en C1-C18, de C1-C18 ramifié, de -CH₂-C₆H₁₀-, de *p*-alkylphénylène, de *p*-phénylène, de *m*-phénylène, de *p*-alkyloxyphénylène, de naphtylène, de -[CH₂CH₂O]ₓ-, de -[CH₂CH₂SOCH₂CH₂]ₓ-, de -[CH₂CH₂SO₂CH₂CH₂]ₓ-, ou de -[NHCHR₄CO]_{y}-, dans lequel x est 1 à 200, y est 1 à 20, et dans lequel R₄ est sélectionné dans le groupe constitué de H-, de Me-, de HSCH₂-, de isopropyle, de but-2-yle, de CH₃SCH₂CH₂-, de benzyle, de 1*H*-indol-3-yl-méthyle, de HOCH₂-, de HOOCCH₂-, de CH₃CH(OH)-, de HOOCCH₂CH₂-, de 4-hydroxybenzyle, de H₂NCOCH₂-, de H₂NCOCH₂CH₂-, de 4-aminobut-1-yle, de 2-guanidinoéthyle, de 1*H*-imidazol-5-yl-méthyle et de 2-méthylprop-1-yle.

6. Radioimmunoconjugué (RIC) pour son utilisation selon l'une quelconque des revendications 4 à 5, dans lequel l'agent chélatant est sélectionné dans le groupe constitué de l'acide diéthylènetriaminepentaacétique (DTPA), de l'acide 1,4,7,10-tétraazacyclododécane-N,N',N",N‴-tétraacétique (DOTA), de l'acide 1,4,8,11-tétraazacyclotétradécane-N,N',N",N‴-tétraacétique (TETA), de l'acide 1,4,7-triazonane-N,N',N"-triacétique (NOTA), de l'acide 2,2'-(2-(((1S,2S)-2-(bis(carboxyméthyl)amino)cyclohexyl)-(carboxyméthyl)amino)éthylazanediyl)diacétique (cyclohexano-DTPA), de l'acide 2,2'-(2-(((1R,2R)-2-(bis(carboxyméthyl)amino)cyclohexyl)-(carboxyméthyl)amino)éthylazanediyl)diacétique, de l'acide 2,2'-(2-(((1S,2R)-2-(bis(carboxyméthyl)amino)cyclohexyl)-(carboxyméthyl)amino)éthylazanediyl)diacétique, de l'acide 2,2'-(2-(((1R,2S)-2-(bis(carboxyméthyl)amino)cyclohexyl)-(carboxyméthyl)amino)éthylazanediyl)diacétique, de l'acide 2,2',2",2‴-(2,2'-(1S,2S)-cyclohexane-1,2-diylbis((carboxyméthyl)azanediyl)bis(éthane-2,1-diyl))bis(azanétriyl)tétraacétique, de l'acide 2,2',2",2‴-(2,2'-(1S,2R)-cyclohexane-1,2-diylbis((carboxyméthyl)azanediyl)bis(éthane-2,1-diyl))bis(azanetriyl)tétraacétique, de l'acide (1*R*)-1-benzyl-diéthylènetriaminepentaacétique, de l'acide (1*S*)-1-benzyl-diéthylènetriaminepentaacétique, de l'acide (2*R*)-2-benzyl-diéthylènetriaminepentaacétique, de l'acide (2*S*)-2-benzyl-diéthylènetriaminepentaacétique, du (2*R*)-2-benzyl-(3*R*)-3-méthyl-DTPA, du (2*R*)-2-benzyl-(3*S*)-3-méthyl-DTPA, du (2*S*)-2-benzyl-(3*S*)-3-méthyl-DTPA, du (2*S*)-2-benzyl-(3*R*)-3-méthyl-DTPA, du (2*R*)-2-benzyl-(4*R*)-4-méthyl-DTPA, du (2*R*)-2-benzyl-(4*S*)-4-méthyl-DTPA, du (2*S*)-2-benzyl-(4*S*)-4-méthyl-DTPA, du (2*S*)-2-benzyl-(4*R*)-4-méthyl-DTPA, du (1*R*)-1-benzyl-(3*R*)-3-méthyl-DTPA, du (1*R*)-1-benzyl-(3*S*)-3-méthyl-DTPA, du (1*S*)-1-benzyl-(3*S*)-3-méthyl-DTPA, du (1*S*)-1-benzyl-(3*R*)-3-méthyl-DTPA, du (1*R*)-1-benzyl-(4*R*)-4-méthyl-DTPA, du (1*R*)-1-benzyl-(4*S*)-4-méthyl-DTPA, du (1*S*)-1-benzyl-(4*S*)-4-méthyl-DTPA, du (1*S*)-1-benzyl-(4*R*)-4-méthyl-DTPA, de l'acide 2,2'-((1R,2R)-2-(((R)-2-(bis(carboxyméthyl)amino)-3-phénylpropyl)(carboxyméthyl)amino)cyclohexylazanediyl)diacétique, de l'acide 2,2'-((1S,2S)-2-(((S)-2-(bis(carboxyméthyl)amino)-3-phénylpropyl)(carboxyméthyl)amino)cyclohexylazanediyl)diacétique, de l'acide 2,2'-((1R,2R)-2-(((S)-2-(bis(carboxyméthyl)amino)-3-phénylpropyl)(carboxyméthyl)amino)cyclohexylazanediyl)diacétique, de l'acide 2,2'-((1S,2S)-2-(((R)-2-(bis(carboxyméthyl)amino)-3-phénylpropyl)(carboxyméthyl)amino)cyclohexylazanediyl)diacétique, de l'acide 2,2'-((1R,2S)-2-(((R)-2-(bis(carboxyméthyl)amino)-3-phénylpropyl)(carboxyméthyl)amino)cyclohexylazanediyl)diacétique, de l'acide 2,2'-((1S,2R)-2-(((S)-2-(bis(carboxyméthyl)amino)-3-phénylpropyl)(carboxyméthyl)amino)cyclohexylazanediyl)diacétique, de l'acide 2,2'-((1S,2R)-2-(((R)-2-(bis(carboxyméthyl)amino)-3-phénylpropyl)(carboxyméthyl)amino)cyclohexylazanediyl)diacétique, de l'acide 2,2'-((1R,2S)-2-(((S)-2-(bis(carboxyméthyl)amino)-3-phénylpropyl)(carboxyméthyl)amino)cyclohexylazanediyl)diacétique, de l'acide (2S)-2-benzyl-1,4,7,10-tétraazacyclododécane-N,N',N",N‴-tétraacétique, de l'acide (2R)-2-benzyl-1,4,7,10-tétraazacyclododécane-N,N',N",N‴-tétraacétique, de l'acide 6-benzyl-1,4,8,11-tétraazacyclotétradécane-N,N',N",N‴-tétraacétique, de l'acide 2-benzyl-1,4,7-triazonane-N,N',N"-triacétique, de l'acide benzyl-3-méthyl-diéthylènetriaminepentaacétique (2B3M-DTPA), de l'acide (R)-2-amino-3-(phényl)propyl)trans-(S,S)-cyclohexane-1,2-diamine-pentaacétique) (Bn-CHX-A"-DTPA) et les sels et dérivés de ceux-ci,
particulièrement l'acide (R)-2-amino-3-(phényl)propyl)trans-(S,S)-cyclohexane-1,2-diamine-pentaacétique) (Bn-CHX-A"-DTPA) et les sels de celui-ci.

7. Radioimmunoconjugué (RIC) pour son utilisation selon l'une quelconque des revendications 1 à 6, dans lequel le radionucléide est lié aux composants de liaison à CD66 via la structure composant de liaison à CD66-NH-CS-NH-Bn-CHX-A"-DTPA.

8. Radioimmunoconjugué (RIC) pour son utilisation selon l'une quelconque des revendications 1 à 7, dans lequel un RIC thérapeutique est administré en une dose ≥ environ 10 MBq/kg de masse maigre (MM), de préférence ≥ environ 15 MBq/kg de MM, de manière davantage préférée ≥ environ 20 MBq/kg de MM, de manière encore davantage préférée ≥ environ 25 MBq/kg de MM, de manière encore davantage préférée ≥ environ 30 MBq/kg de MM et de manière encore davantage ≥ environ 35 MBq/kg de MM, jusqu'à 60 MBq/kg de MM ou jusqu'à 45 MBq/kg de MM.

9. Radioimmunoconjugué (RIC) pour son utilisation selon l'une quelconque des revendications 2 à 8, dans lequel le traitement de maladies associées à la moelle osseuse précisément de l'AL-amyloïdose comprend en outre la transplantation de cellules souches, particulièrement la transplantation de cellules souches autologues ou allogéniques, plus particulièrement la transplantation de cellules souches autologues, particulièrement dans lequel
le RIC thérapeutique est destiné à être administré 6 à 16 jours, de préférence 6 à 12 jours, avant la transplantation de cellules souches et/ou
dans lequel le traitement de maladies associées à la moelle osseuse telles que l'AL-amyloïdose n'implique pas l'administration d'un agent antitumoral supplémentaire, tel que le melphalan, ou d'un agent immunosuppresseur au moins quatre semaines avant et/ou après la transplantation de cellules souches.

10. Radioimmunoconjugué (RIC) pour son utilisation selon l'une quelconque des revendications 2 à 10, dans lequel le RIC thérapeutique est destiné à être administré à un patient qui a été traité avec du melphalan à haute dose suivi par une transplantation de cellules souches mais dont la maladie a progressé et/ou est destiné à être administré à un patient qui a été traité avec une thérapie d'induction mais dont la maladie a progressé.

11. Radioimmunoconjugué (RIC) pour son utilisation selon l'une quelconque des revendications 2 à 11, dans lequel le RIC thérapeutique est destiné à être administré à un patient qui n'est pas éligible à un traitement avec du melphalan HD avant une HSCT.

12. Radioimmunoconjugué (RIC) pour son utilisation selon l'une quelconque des revendications 2 à 11, dans lequel le traitement de maladies associées à la moelle osseuse, précisément de l'AL-amyloïdose, comprend les étapes de
(a) l'administration facultative d'un RIC d'imagerie à un patient, notamment 2 à 10 jours avant l'étape (b), dans lequel le RIC d'imagerie comprend particulièrement un radionucléide indium-111 (¹¹¹IN),
(b) l'administration d'un RIC thérapeutique au patient, notamment 6 à 16 jours avant l'étape (c), et
(c) la transplantation de cellules souches autologues ou allogéniques.

13. Radioimmunoconjugué (RIC) pour son utilisation selon les revendications 1, 3, 4 à 8 et 11, dans lequel la transplantation de cellules souches est autologue ou allogénique.
